# FASCICULE DE BREVET EUROPEEN

(11) **EP 2 867 204 B1**
(45) Date de publication et mention de la délivrance du brevet: **14.09.2016**
(21) Numéro de dépôt: 13744661.3
(22) Date de dépôt: 28.06.2013
(51) Int. Cl.: C07D 207/12, A61K 31/401, A61P 25/00

(54) **NOUVEAUX DERIVES DE PYRROLIDINE**
NEUARTIGE PYRROLIDINDERIVATE
NOVEL PYRROLIDINE DERIVATIVES

(30) Priorité: 02.07.2012 FR 1256322
(43) Date de publication de la demande: 06.05.2015
(73) Titulaire: Université Claude Bernard Lyon I, 69622 Villeurbanne Cedex (FR); Institut National des Sciences Appliquées de Lyon, 69621 Villeurbanne (FR); Centre National de la Recherche Scientifique, 75794 Paris Cedex 16 (FR); CPE Lyon FCR, 69100 Villeurbanne (FR)
(72) Inventeur: PRALY, Jean-Pierre, 69390 Vourles (FR); AOUADI, Kaïss, Le Kef 7100 (TN); CECIONI, Samy, 69600 Oullins (FR); DENOROY, Luc, 69100 Villeurbanne (FR); PARROT, Sandrine, 69100 Villeurbanne (FR)
(74) Mandataire: Sarlin, Laure V.
(86) Numéro de dépôt international: PCT/FR2013/051524
(87) Numéro de publication internationale: WO 2014/006307

(56) Documents cités:
- US-A- 5 888 996
- US-A1- 2004 259 917

## Description

La présente invention concerne le domaine technique du traitement des maladies du système nerveux central. Plus précisément, l'invention a pour objet des nouveaux dérivés de pyrrolidine, les compositions pharmaceutiques contenant de tels composés, et de tels composés, en tant que médicament, et notamment pour leur utilisation dans le traitement, en particulier chez les êtres humains, de l'épilepsie, de l'ischémie, d'une maladie neurodégénérative telle que la maladie de Parkinson ou la chorée de Huntington, la sclérose en plaques, la maladie de Devic, la maladie d'Alzheimer, d'une maladie psychiatrique telle que la schizophrénie, la dépression, l'addiction, l'allodynie, l'hyperalgie, de la douleur (analgésie), d'un cancer d'un organe ou tissu périphérique, de l'obésité, d'un trouble squeletto-musculaire, d'une maladie cardio-vasculaire ou d'une maladie des voies digestives ou pour le contrôle de la prise alimentaire.

L'acide glutamique (Glu) et, dans une moindre mesure, l'acide aspartique (Asp) sont les neurotransmetteurs majeurs du centre nerveux central **[1-2].** Ces deux acides aminés neurotransmetteurs sont présents dans tous les réseaux neuronaux et présentent une distribution anatomique ubiquitaire dans le cerveau. Ils jouent un rôle dans les processus neurophysiologiques comme le contrôle du système autonome, les fonctions motrices, le contrôle de la douleur, les tâches cognitives, l'apprentissage et la mémoire, .... Des expériences menées sur des modèles animaux de maladies humaines et des données obtenues chez l'homme ont révélé des altérations de ce type de neurotransmission dans des pathologies comme l'épilepsie, l'ischémie, des maladies neurodégénératives (Parkinson, Huntington) ou des maladies psychiatriques comme la schizophrénie **[2-6].** Ces quatre grands thèmes de recherche font classiquement l'objet d'études du contrôle des neurones acido-aminergiques.

D'autres pathologies sont également connues, comme impliquant le glutamate : on peut notamment citer la sclérose en plaques, la maladie de Devic, la maladie d'Alzheimer, la dépression, mais aussi l'addiction, l'allodynie et l'hyperalgie **[7].**

Il existe deux grandes familles de récepteurs (ionotropes et métabotropes) de l'acide glutamique et de l'acide aspartique. Ces récepteurs ont été classés en 5 types (kainate et NMDA pour les ionotropes, groupe I, groupe II et groupes III pour les métabotropes) et leurs sous-types respectifs ont été déterminés en fonction de leurs affinités, des couplages intracellulaires et/ou de leur localisation anatomique (le glutamate agit sur tous les sous-types, alors que l'aspartate n'agit que sur les récepteurs NMDA). Les acides aminés excitateurs restent un enjeu primordial dans la recherche de cibles pharmacologiques visant sélectivement un ou des sous-types de leurs récepteurs. Par exemple, des agents pharmacologiques ciblant la transmission excitatrice des acides aminés ont été développés pour traiter des épilepsies ou empêcher la toxicité induite par une ischémie. De nombreux anti-épileptiques commerciaux ont une structure amino-acide. A titre d'exemple, on peut citer le phénobarbital, la primidone, la phénytoine, parmi les plus anciennes et l'acétazolamide, la zonisamide et la rufinamide parmi les plus récents. Des antiépileptiques analogues du Gaba et dérivés de la pyrrolidin-2-one sont également les principes actifs de médicaments commerciaux pour le traitement de l'épilepsie.

On peut citer l'éthosuximide, le piracetam, l'oxiracetam, le levetiracetam, le pramiracetam, l'aniracetam et le nefiracetam.

D'autres dérivés de pyrrolidin-2-one sont également connus pour d'autres activités.

L'acide penmacrique des graines de Pentachléthra macrophylla est décrit pour son usage alimentaire et médicinal **[8],** l'alahopsine est un antibiotique utilisé contre diverses bactéries Gram positives et négatives et aussi un inhibiteur de la propyl collagen hydroxylase **[9],** tout comme la dealanylalahopsin **[9,10].**

US 5,888,996 divulgue des stéroïdes en tant que modulateurs de NMDA utiles pour le traitement de maladie du système nerveux central.

US 2004/0259917 divulgue des dérivés d'imidazole en tant que modulateurs mGlu5 utiles pour le traitement de maladie du système nerveux central.

Néanmoins, il existe toujours un besoin pour des nouveaux composés présentant un intérêt pour le traitement de maladies du système nerveux central, et en particulier pour des nouveaux composés qui présentent une activité modulatrice des taux de glutamate et d'aspartate extracellulaires.

Les inventeurs de la présente demande de brevet se sont intéressés à d'autres dérivés de la pyrrolidine, autres que des dérivés de la pyrrolidin-2-one.

Dans ce contexte, la présente invention concerne des dérivés de pyrrolidine de formule (I) : 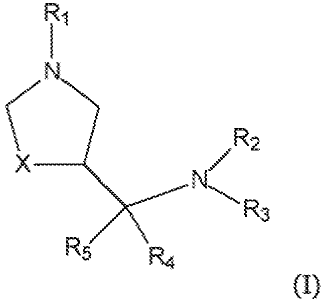 dans laquelle :
- R₁ représente un atome d'hydrogène, un groupe (C₁-C₆)alkyle ou C(O)(C₁-C₆)alkyle,
- R₂ et R₃ identiques ou différents, représentent, chacun indépendamment l'un de l'autre, un atome d'hydrogène ou un groupe (C₁-C₆)alkyle ; ou bien R₂=H et R₃= C(O)(C₁-C₆)alkyle,
- R₄ représente -COOH, -CN, -COORₐ, -C(=NOH)NH₂, -CH₂NH₂, -CH₂OH, -C(O)NH₂, -C(O)NHRₐ, -COSRₐ, avec Rₐ qui représente un groupe (C₁-C₆)alkyle, ou bien R₄ représente un groupe tétrazole ou 1,2,4-oxadiazole,
- R₅ représente un atome d'hydrogène ou un groupe (C₁-C₆)alkyle,
- X représente -C(O)- ou -CHR_{b}-, avec R_{b} qui représente -OR_{c} ou -OC(O)R_{c}, R_{c} représentant un atome d'hydrogène ou un groupe (C₁-C₆)alkyle,
éventuellement sous une forme zwitterionique,
sous la forme d'un isomère optique pur, ou sous la forme d'un mélange d'isomères optiques selon toutes proportions, ou sous une forme enrichie en un isomère optique, ainsi que leurs sels pharmaceutiquement acceptables, solvats ou hydrates.

Dans la définition des composés selon l'invention, par « groupe alkyle », on entend une chaîne hydrocarbonée, saturée, linéaire ou ramifiée. A titre d'exemple de groupe alkyle comprenant de 1 à 6 atomes de carbone, nommés (C₁-C₆)alkyle, on peut citer, notamment, les groupes méthyle, éthyle, *n*-propyle, *iso*-propyle, *n*-butyle, *tert-*butyle, *sec*-butyle, n-pentyle, n-hexyle.

Selon des modes de réalisation préférés, X représente -CHR_{b}, avec notamment R_{b}=OH. Dans ce cas, les dérivés de pyrrolidine selon l'invention peuvent répondre notamment à la formule (Ia) : 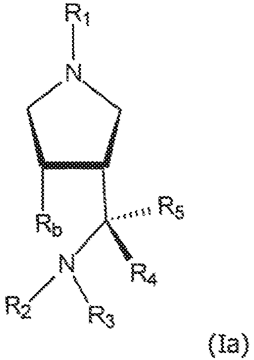 dans laquelle R₁, R₂, R₃, R₄, R₅ et R_{b} sont tels que définis pour les composés de formule (I), éventuellement sous la forme d'un sel pharmaceutiquement acceptable, solvat ou hydrate, ou à la formule (Ib) : 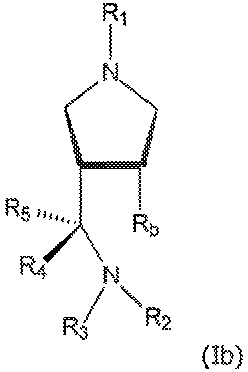 dans laquelle R₁, R₂, R₃, R₄, R₅ et R_{b} sont tels que définis pour les composés de formule (I), éventuellement sous la forme d'un sel pharmaceutiquement acceptable, solvant ou hydrate,

Selon des modes de réalisation particuliers, les composés de formule (I), (Ia) ou (Ib), présentent une caractéristique ci-dessous ou une combinaison des caractéristiques ci-dessous, voire toutes les caractéristiques ci-dessous :
- R₁ est un atome d'hydrogène ;
- R₂=H et R₃ représente un atome d'hydrogène ou un groupe méthyle ou -C(O)CH₃;
- R₄ représente -COOH, -C(O)NHCH₃ ou -CH₂NH₂;
- R₅ est un atome d'hydrogène,
- Il se présente sous une forme salifiée ou zwitterionique comportant un ammonium quaternaire.

Les composés utilisés dans le cadre de l'invention sont préparés selon des techniques classiques. Ils peuvent, notamment être obtenus selon des procédés analogues à ceux utilisés dans les exemples.

Il est possible d'utiliser soit une approche dite chirale, soit une synthèse dite achirale, comme explicité ci-dessous,

Le SCHEMA 1 ci-dessous, dans lequel R'₁ représente R₁ tel que défini pour (I), ou un groupe protecteur du type benzoyle, illustre l'approche chirale,

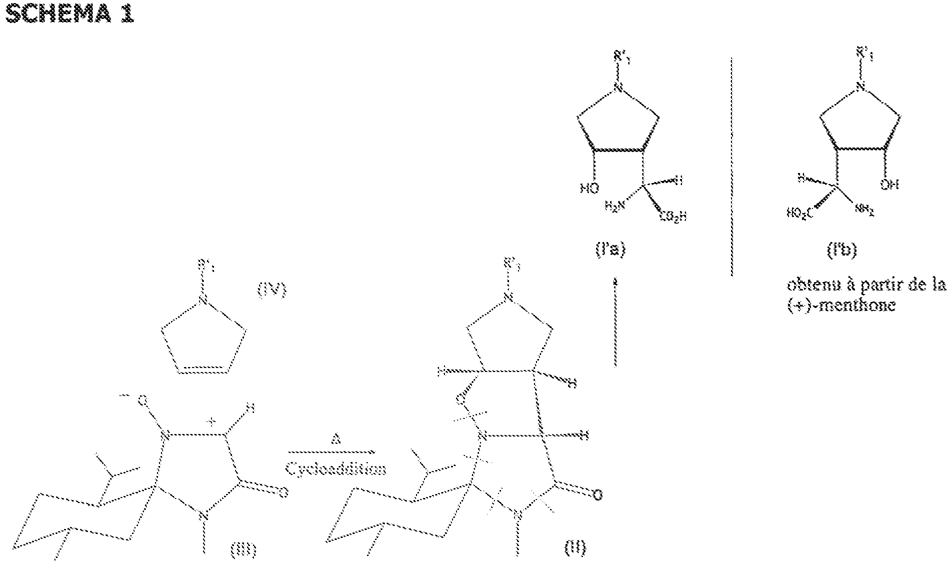

Dans une première étape, on réalise une cycloaddition, notamment à une température proche de 110 °C, éventuellement sous activation aux micro-ondes, de la nitrone (III) et d'un alcène (IV) pour conduire à un cycloadduit (II) qui, après une étape d'hydrogénolyse sous action d'hydrogène en présence d'un catalyseur (par exemple du palladium sur charbon) entraînant le clivage de la liaison N-O, et une hydrolyse acide (de préférence, avec un mélange anhydride et acide acétique acidifié avec H₂SO₄) puis basique, entrainant le clivage des trois liaisons au niveau de la nitrone comme illustré sur le **SCHEMA 1** ci-dessus, donne les composés (I'a) ou (I'b). Les composés (I'a) et (I'b) correspondent directement à des composés de formule (Ia) ou (Ib) ou peuvent être fonctionnalisés au niveau du groupe amino ou du groupe acide carboxylique selon des techniques bien connues de l'homme de l'art.

La fonction NR'₁ elle aussi pourra être déprotégée ou alkylée, pour conduire aux composés souhaités, selon des méthodes classiques et la fonction OH pourra être alkylée pour conduire à un groupe -O(C₁-C₆)alkyle.

Une autre stratégie pourra être d'adapter, comme dans les exemples, les conditions de clivage pour aboutir directement aux groupements -NR₂R₃ et/ou R₄ souhaité(s).

Les nitrones (III) peuvent être obtenues à partir des menthones (IV) chirales selon le **SCHEMA 2** ci-dessous.

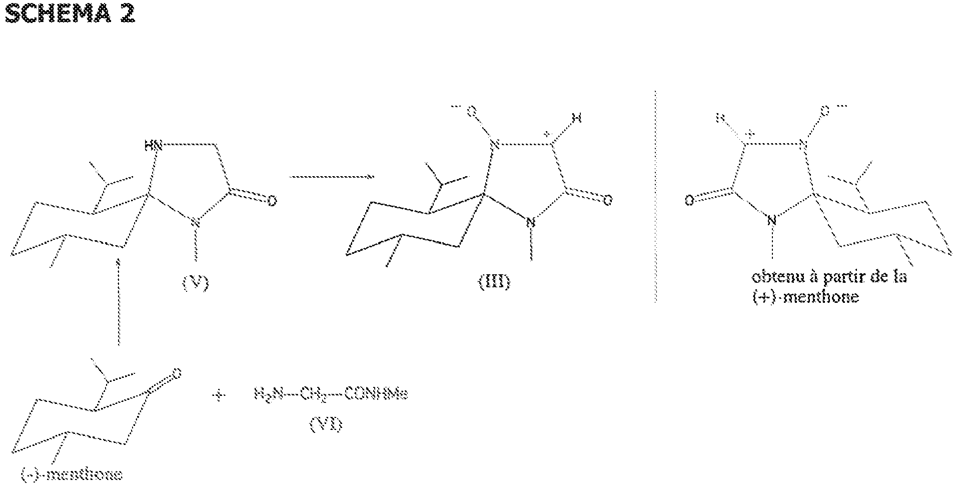

L'introduction de groupement R₅ différent de l'hydrogène peut se faire ultérieurement.

Les nitrones (III) peuvent être obtenues à partir des composés (V) en présence d'acide méta-chloro-perbenzoïque m-CPBA (de préférence 2 équivalents) et de l'acide 3-chlorobenzoique (qui est un sous-produit de la réaction d'oxydation). Les réactifs de départ sont disponibles dans le commerce (notamment la 3-pyrroline et la N-benzyl-3-pyrroline sont commerciales) ou facilement accessibles. Les composés (VI) peuvent notamment être obtenus à partir de l'ester correspondant par réaction avec la méthylamine sur le chlorhydrate de l'ester méthylique de la glycine, à une température de l'ordre de 0 à 78 °C, et notamment à température ambiante.

Dans l'approche achirale, il est possible d'utiliser soit des nitrones, soit des oxydes de nitrile dépourvus de chiralité. De nombreuses nitrones achirales, équivalentes de glycine sont disponibles dans la littérature. Leur synthèse repose, notamment, sur la formation de cyanométhylamines substituées, à partir d'halogénoacétonitrile et d'amines substituées, suivie d'une oxydation en nitrones **[11].**

Une autre voie d'accès à des nitrones appropriées fait appel à des hydroxylamines réagissant sur la fonction carbonyle de l'acide glyoxylique (commercial, soit pur monohydraté, soit en solution aqueuse), ou de ses esters commerciaux (éthyl) ou faciles à préparer (isopropyl) **[12, 13, 14, 15, 16, 17, 18, 19, 20, 21, 22],**

Il est également possible, dans l'approche achirale, de réaliser la cycloaddition, non pas avec une nitrone mais avec un oxyde de nitrile. De tels oxydes de nitrile peuvent être obtenus à partir des aldéhydes correspondants et peuvent ensuite être utilisés dans des cycloadditions dipolaires qui, sur des alcènes, donnent des isoxazolines. Par réduction, on obtient une coupure du lien N-O et simultanément une réduction de la liaison double **[23]** selon le **SCHEMA 3** ci-dessous dans lequel R'₁ représente R₁ tel que défini pour (I), ou un groupe protecteur du type benzoyle ou tert-butoxycarbonyle (boc).

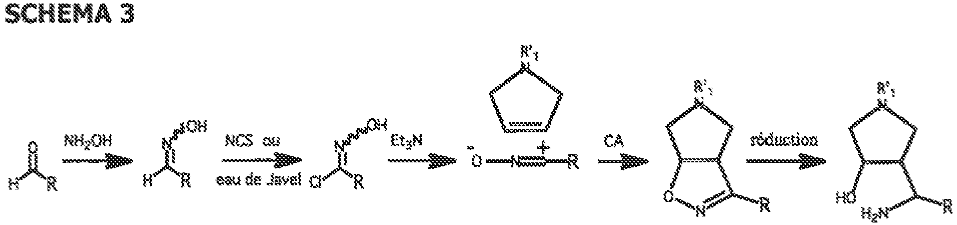

Les composés selon l'invention peuvent, notamment, présenter deux ou trois carbones asymétriques. Leurs différents isomères optiques font partie intégrante de l'invention. Les différents composés selon l'invention peuvent donc se trouver sous toutes les formes isomériques possibles, éventuellement en mélange selon toutes proportions, à moins qu'il n'en soit spécifié autrement. Selon un mode de réalisation particulier, les composés selon l'invention se trouvent sous une forme racémique, les deux énantiomères se trouvant en proportions sensiblement égales. Selon un autre mode de réalisation, les composés de formule (I) de l'invention peuvent se trouver sous une forme enrichie en un diastéréoisomère ou énantiomère, avec un excès diastéréoisomérique ou énantiomérique supérieur à 80 %, voire même supérieur à 95%, voire sous une forme isomérique pure c'est-à-dire avec un excès diastéréoisomérique ou énantiomérique supérieur à 99 %, voire même égal à 100%.

Les composés (I) peuvent être isolés sous forme d'isomères ou sous une forme enrichie en un diastéréoisomère ou énantiomère par les techniques classiques de séparation : on pourra utiliser, par exemple des recristallisations fractionnées d'un sel du racémique avec un acide ou une base optiquement active dont le principe est bien connu ou, le plus souvent, les techniques classiques de chromatographies sur phase chirale ou non chirale. Il est également possible comme expliqué ci-dessus d'utiliser directement une synthèse dite chirale conduisant à isomère optique désiré.

Les composés de formule (I) ci-dessus comprennent également ceux dans lesquels un ou plusieurs atomes d'hydrogène, de carbone ou d'azote ont été remplacés par des halogènes, notamment de chlore ou de fluor y compris par leur isotope radioactif par exemple le tritium, le carbone-14, ou le fluor-18. De tels composés marqués sont utiles dans des travaux de recherche, de métabolisme ou de pharmacocinétique, dans des essais biochimiques,

Les groupes fonctionnels éventuellement présents dans la molécule des composés de formule (I) et dans les intermédiaires réactionnels peuvent être protégés au cours des synthèses, soit sous forme permanente soit sous forme temporaire, par des groupes protecteurs qui assurent une synthèse univoque des composés attendus.

Les réactions de protection et déprotection sont effectuées selon des techniques bien connues de l'homme de l'art. Par groupe protecteur temporaire des amines, alcools ou des acides carboxyliques on entend les groupes protecteurs tels que ceux décrits dans Protective Groups in Organic Synthesis, Greene T.W. et Wuts P.G.M., ed John Wiley et Sons, 2006 et dans Protecting Groups, Kocienski P.J., 1994, Georg Thieme Verlag.

Les sels des composés selon l'invention sont préparés selon des techniques bien connues de l'homme de l'art. Les sels des composés de formule (I) selon la présente invention comprennent ceux avec des acides ou des bases, en fonction des substituants présents. Ces acides ou bases peuvent être choisis parmi les acides et bases minéraux et organiques qui permettent une séparation ou une cristallisation convenable des composés de formule (I), ainsi que des sels pharmaceutiquement acceptables. En tant qu'acide approprié, on peut citer l'acide oxalique ou un acide optiquement actif, par exemple un acide tartrique, un acide dibenzoyltartrique, un acide mandélique ou un acide camphorsulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le para-toluènesulfonate, le mésylate, le bésylate, l'isotionate.

En tant que base appropriée, on peut citer : la lysine, l'arginine, la méglumine, la bénéthamine, la benzathine et celles qui forment des sels physiologiquement acceptables, tels que des sels de sodium, de potassium, de calcium.

Comme composés sous forme hydratée, on peut citer, à titre d'exemple, les semi-hydrates, monohydrates et polyhydrates.

Par solvat, on entend une forme du composé associé à une ou plusieurs molécules de solvant, notamment utilisé lors de sa synthèse ou lors de sa purification, sans pour autant être en solution dans ce dernier.

Dans le cadre de l'invention, il a été mis en évidence que les composés de formule (I) sous la forme d'un mélange d'isomères optiques selon toutes proportions, ou sous une forme enrichie en un isomère optique, ainsi que leurs sels pharmaceutiquement acceptables, soldats ou hydrates présentent des effets immédiats et significatifs sur les taux de glutamate et d'aspartate extracellulaires. L'activité des composés selon l'invention correspond à une augmentation ou à une diminution des taux en glutamate et en aspartate selon les composés. Ces effets sont d'ailleurs très dépendants des taux de glutamate endogène, comme cela a été montré pour le kainate, par exemple. En particulier, la plupart des composés selon l'invention, et les composés de formule (I.1) à (I.4) explicités dans les exemples, entrainent une diminution des taux de glutamate extracellulaire dans des cerveaux de rats, lorsque le taux de glutamate initial est élevé, notamment supérieur à 10 µmol/L. Ils peuvent donc être utiles pour le traitement de pathologies correspondant à une hyperactivité glutamatergique. En extrapolant à partir des concentrations utilisées lors de l'introduction des composés (I.1) à (I.4) par dialyse inverse, la gamme d'activité après administration périphérique est estimée entre 5 et 100 mg/kg chez l'animal.

La présente invention a également pour objet les composés tels que définis précédemment, pour leur utilisation en tant que médicament, et, en particulier, pour leur utilisation en tant que médicament pour le traitement de l'épilepsie, de l'ischémie, d'une maladie neurodégénérative telle que la maladie de Parkinson ou la chorée de Huntington, la maladie d'Alzheimer, la sclérose en plaques, la maladie de Devic, d'une maladie psychiatrique telle que la schizophrénie, la dépression, l'addiction, l'allodynie, l'hyperalgie, de la douleur (analgésie), d'un cancer d'un organe ou tissu périphérique, de l'obésité, d'un trouble squeletto-musculaire, d'une maladie cardio-vasculaire ou d'une maladie des voies digestives ou pour le contrôle de la prise alimentaire.

Dans le cadre de l'invention, l'utilisation des composés selon l'invention, dans le traitement des êtres humains est particulièrement visée.

Les compositions pharmaceutiques comprenant un composé tel que précédemment défini, en association avec au moins un excipient pharmaceutiquement acceptable, font également partie intégrante de l'invention.

Le terme « traitement » désigne toute mesure thérapeutique prophylactique ou suppressive d'une maladie ou désordre conduisant à un effet clinique souhaitable ou à tout effet bénéfique, incluant notamment la suppression ou la diminution d'un ou plusieurs symptômes, la régression, le ralentissement ou la cessation de la progression de la maladie ou du désordre qui y est associé.

Par « quantité thérapeutiquement efficace », on désigne toute quantité d'une composition qui améliore un ou plusieurs des paramètres caractéristiques de l'affection traitée.

De par leur activité, les composés selon l'invention, quelle que soit leur forme isomérique, peuvent être utilisés pour la fabrication d'un médicament destiné à traiter l'épilepsie, l'ischémie, une maladie neurodégénérative telle que la maladie de Parkinson ou la chorée de Huntington, la maladie d'Alzheimer, la sclérose en plaques, la maladie de Devic, une maladie psychiatrique telle que la schizophrénie, la dépression, l'addiction, l'allodynie, l'hyperalgie, la douleur (analgésie), un cancer d'un organe ou tissu périphérique, l'obésité, un trouble squeletto-musculaire, une maladie cardio-vasculaire ou une maladie des voies digestives ou d'un médicament destiné au contrôle de la prise alimentaire.

En particulier, les composés selon invention trouveront un intérêt particulier pour leur utilisation dans le traitement de la maladie de Parkinson, l'épilepsie, les troubles addictifs ou l'ischémie,

La présente invention a également pour objet les compositions pharmaceutiques administrables aux animaux et, en particulier à l'homme, contenant une dose efficace d'un composé selon l'invention, et un ou des excipients convenables selon notamment la Pharmacopée Européenne 7^{ème} édition. L'invention concerne donc les compositions pharmaceutiques comprenant un composé selon l'invention, avec au moins un excipient pharmaceutiquement acceptable.

Les excipients présents dans les compositions pharmaceutiques selon l'invention sont choisis selon la forme pharmaceutique et le mode d'administration souhaité. Dans les compositions pharmaceutiques de la présente invention pour l'administration orale, sublinguale, sous-cutanée, intramusculaire, intra-veineuse, intracartilage, topique, intratrachéale, intranasale, transdermique, rectale ou intraoculaire, les composés selon l'invention peuvent être administrés sous formes unitaires d'administration, en mélange avec des supports pharmaceutiques classiques, aux animaux et/ou aux êtres humains pour la prophylaxie ou le traitement des maladies ci-dessus. Les formes unitaires d'administration appropriées comprennent les formes par voie orale, telles que les comprimés, les gélules, les poudres, les granules et les solutions ou suspensions orales, les formes d'administration sublinguale, buccale, intra trachéale, intra nasale, les formes d'administration sous-cutanée, intramusculaire, intra cartilage ou intraveineuse et les formes d'administration rectale. Pour l'application topique, on peut utiliser les composés selon l'invention dans des crèmes, pommades, patches ou lotions.

Afin d'obtenir l'effet souhaité, le composé selon l'invention sera présent dans la composition à une dose thérapeutiquement efficace, en particulier pour le traitement des êtres humains. La dose de principe actif varie, par exemple, entre 1 et 50 mg par kg de poids du corps du patient traité et par jour.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les compositions pharmaceutiques contenant un composé selon l'invention, peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Une préparation sous forme de sirop ou d'élixir ou pour l'administration sous forme de gouttes peut contenir ingrédient actif conjointement avec un édulcorant acalorique, un antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

D'une façon générale, les mêmes variantes que celles indiquées précédemment pour les composés (I) sont applicables *mutatis mutandis* aux médicaments, compositions et utilisations mettant en oeuvre ces composés.

Les exemples ci-après permettent d'illustrer l'invention, mais n'ont aucun caractère limitatif,

### I. Synthèses chimique:

L'attribution des signaux de RMN suit la numérotation indiquée ci-après pour les molécules **3, 4, 8,** et **11.**

La préparation des composés (I.1) à (I.4) est présentée sur **le SCHEMA 4** ci-après.

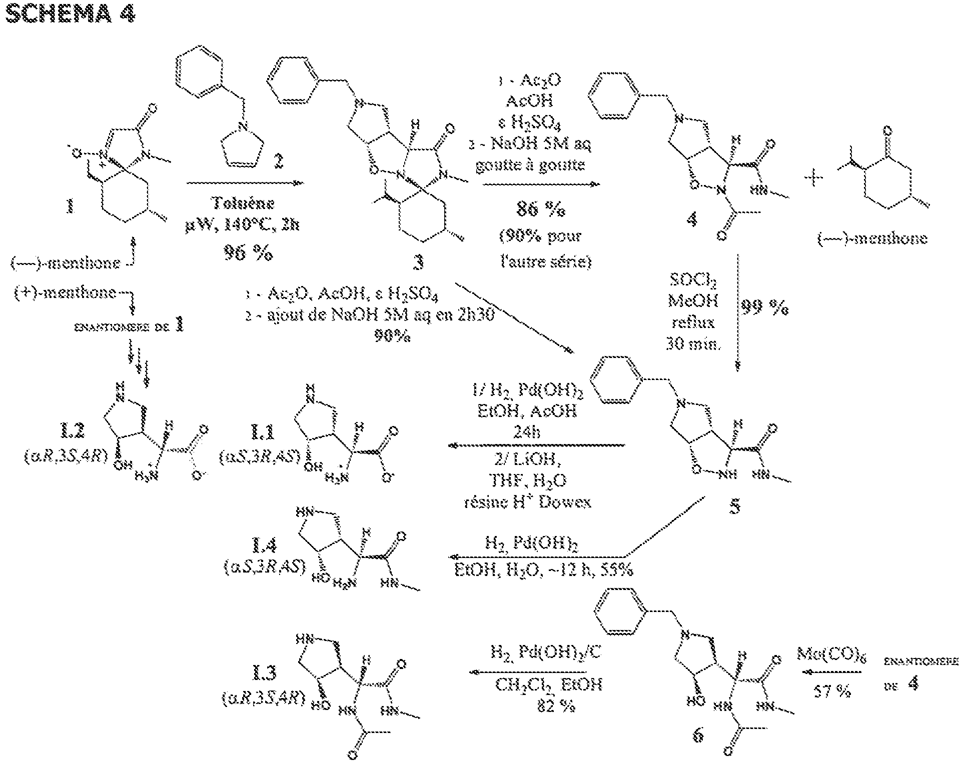

### PREPARATION DU COMPOSE I.1 : Acide 3-pyrrolidine acétique, α-amino, 4-hydroxy (αS,3R,4S) ou acide α-amino-(4-hydroxy-pyrrolidin-3-yl)acétique (αS,3R,4S)

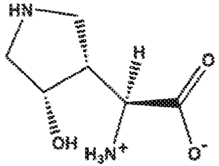

Cycloaddition de la nitrone **1** (correspondant aux composés de formule (III)) sur la *N*-Bn-3-pyrroline commerciale **2** (correspondant aux composés de formule (IV)): synthèse de **3** (correspondant aux composés de formule (II)):

La nitrone **1** (392 mg, 1,64 mmol) **[24, 25]** et la *N*-Bn-3-pyrroline commerciale **2** en excès (314 mg, 1,97 mmol, 1,2 éq) sont introduites dans un flacon adapté à un réacteur à microondes Biotage Initiator. Après que le flacon ait été rempli d'argon, 2,5 mL de toluène anhydre y sont coulés. Le flacon scellé avec un septum et monté 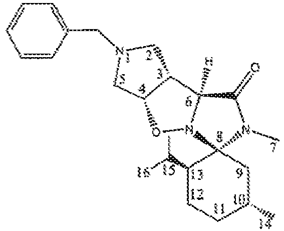 dans l'appareil microonde est irradié avec la consigne de maintenir une température de 140°C sur 2 h pour convertir totalement la nitrone **1.** Une fois le flacon refroidi, le brut réactionnel est concentré puis purifié par flash chromatographie sur colonne de silice (acétate d'éthyle) pour conduire au cycloadduit **3** (625 mg, 1,57 mmol) avec un rendement de 96 % et une stéréosélectivité totale (la réaction a été menée sur une échelle de 5 grammes avec des résultats similaires).

Des monocristaux du composé **3** ont été obtenus à partir d'une solution de diéthyl éther saturée en **3,** laissée au froid (freezer).

***R_{f} =*** 0.48 (EtOAc). **[α]_{D}** = + 40,4 (*c* 1.1, CH₂Cl₂). **RMN ¹H (400 MHz, CDCl₃) :** *δ* 7.37 - 7.20 (m, 5H, CH-ar), 4.59 (td, *J* = 7.0 Hz, *J* = 3.0 Hz, 1H, H-4), 3.71 - 3.49 (m, 3H, NC*H₂*Ph, H-6), 3.42 (dd, *J* = 10.3 Hz, *J* = 6.6 Hz, 1H, H-3), 2.78 (dd, *J* = 10.3 Hz, *J =* 3.0 Hz, 1H, H-5), 2.75 - 2.69 (m, 4H, NC*H₃*, H-2), 2.65 (dd, *J* = 9.4 Hz, *J* = 3.7 Hz, 1H, H-2'), 2.58 (dd, *J* = 9.9 Hz, *J* = 6.6 Hz, 1H, H-5'), 2.14 - 2.08 (m, 1H, H-9), 2.00 (dtt, *J* = 12.9 Hz, *J* = 6.5 Hz, *J* = 3.3 Hz, 1H, H-10), 1.90 - 1.78 (m, 2H, H-11, H-12), 1.68 - 1.59 (m, 1H, H-12'), 1.48 (dt, *J* = 13.5 Hz, *J* = 6.7 Hz, 1H, H-15), 1.38 (dd, *J =* 12.1 Hz, *J =* 3.2 Hz, 1H, H-13), 1.18 (t, *J* = 12.3 Hz, 1H, H-9'), 0.95 - 0.85 (m, 10H, H-11', H-14, H-16) ppm. **RMN ¹³C (100 MHz, CDCl₃) :** *δ* 172.8 (C=O), 138.9 (C^{IV}-ar), 128.6 (CH-ar), 128.3 (CH-ar), 127.1 (CH-ar), 88.0 (C-8), 79.6 (C-4), 71.9 (C-6), 59,6 (N*C*H₂Ph), 59,4 (C-5), 59.3 (C-2), 49.1 (C-3), 48.2 (C-13), 41.0 (C-9), 35.0 (C-11), 29.0 (C-10), 25.9 (N*C*H₃), 24.5 (C-15), 24.2 (*C*H₃), 22.6 (C-12), 22.4 (*C*H₃), 18.7 (*C*H₃) ppm. **HR-ESI-QToF MS** (mode positif): *m*/*z* calc pour C₁₄H₃₆N₃O₂ [M + H]⁺ 398.2802, trouvé 398.2806.

### Acétolyse du composé 3 en hétérobicycle 4 :

L'acétolyse du composé **3** a lieu par addition de quelques gouttes (∼ 6 gouttes) d'acide sulfurique concentré (H₂SO₄ à 95 %) à un mélange réactionnel contenant **3** (226 mg, 0,57 mmol), de l'acide acétique (3 mL) et de l'anhydride acétique (3 mL), puis chauffage du milieu à 60 °C pendant 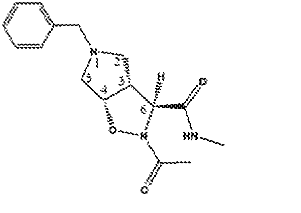 6h30 sous argon. Une fois revenu à température ambiante, le milieu est refroidi au bain de glace à 0 °C, puis les espèces acides sont neutralisées par une solution de soude 5M (ajout lent avec contrôle du pH). Lorsque le pH est d'environ 8, le milieu est versé dans une solution saturée de bicarbonate de sodium NaHCO₃ (300 mL) puis une extraction liquide-liquide (dichlorométhane, 5 x 50 mL) est réalisée. Après concentration et séchage, le produit brut est purifié par flash chromatographie sur silice (acétate d'éthyle) pour donner la molécule **4** (148 mg, 0,49 mmol) avec un rendement de 86 % (90 % pour l'énantiomère ***ent-4***). Parmi les fractions éluées en tête, l'auxiliaire chiral (-)-menthone **1** peut être récupéré et réutilisé (taux de récupération de 80 % sur l'hydrolyse de **3** en **4** plus menthone).

***R_{f}*=** 0.28 (EtOAc). **[α]_{D} = -** 14.2 (*c*1, CH₂Cl₂). **RMN ¹H (400 MHz, CDCl₃) :** *δ* 7.34 - 7.20 (m, 5H, CH-ar), 6.44 (bs, 1H, N*H*CH₃), 4.97 (bs 1H, H-6), 4.71 (dd, *J =* 7.1 Hz, *J* = 4.8 Hz, 1H, H-4), 3.62 (t, *J =* 7.1 Hz, 1H, H-3), 3.57 (d, *J* = 12.7 Hz, 1H, ½ NC*H*₂Ph), 3.33 (d, *J =* 12.7 Hz, 1H, ½ N*C*H₂Ph), 3.10 - 2.99 (m, 2H, H-2, H-5), 2.79 (d, *J* = 4.9 Hz, 3H, NC*H*₃), 2.34 (dd, *J* = 10.0 Hz, *J* = 7.1 Hz, 1H, H-2'), 2.19 (dd, *J* = 11.6 Hz, *J* = 4.8 Hz, 1H, H-5'), 2.09 (s, 3H, NC(O)CH₃) ppm. **RMN ¹³C (100 MHz, CDCl₃)** : *δ* 175.7 (MeN*C*=O), 169.8 (*C*(O)NHCH₃), 138.1 (C^{IV}-ar), 128.9 (CH-ar), 128.5 (CH-ar), 127.5 (CH-ar), 85.0 (C-4), 66.2 (C-6), 60.7 (C-5), 60.4 (C-2), 59.9 (N*C*H₂Ph), 48.4 (C-3), 26.4 (N*C*H₃), 21.2 (C(O)*C*H₃) ppm. **HR-ESI-QToF MS** (mode positif): m/z calc pour C₁₆H₂₂N₃O₃ [M + H]⁺ 304.1656, trouvé 304.1667.

### N-Déacétylation de 4 en 5 :

Pour faciliter la coupure de la liaison N-O, il est préférable que l'atome d'azote soit déacétylé. La *N*-déacétylation est obtenue en ajoutant **4** (122 mg, 0,40 mmol) dissous dans 4 mL de dichlorométhane à une solution de chlorure de 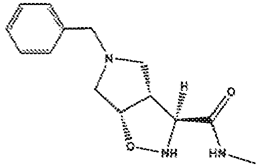 thionyle SOCl₂ (151 µL, 5,2 éq) dans le méthanol fraîchement distillé (3 mL), la solution étant préalablement agitée 10 min à 0°C (libération d'acide chlorhydrique anhydre). Après 15 min à 0°C, le milieu est laissé revenir à température ambiante, puis on chauffe au reflux pendant 1 h. Après refroidissement et addition d'une solution de carbonate de sodium Na₂CO₃ à 5% (4 mL), la phase aqueuse est extraite à l'acétate d'éthyle (2 x 30 mL). La phase organique est à nouveau lavée avec une solution de carbonate de sodium à 5% (2 x 40 mL), avec re-extraction de la phase aqueuse au dichlorométhane (2 x 20 mL). L'évaporation des solvants et la lyophilisation donnent le composé **5** (104 mg, 0.4 mmoi, 99 %) soit quantitativement.

***R_{f}*** = 0.74 (CH₂Cl₂/IPA, 1/1). **[α]_{D}** = + 10.9 (*c* 1.3, CH₂Cl₂), **RMN ¹H (400 MHz, CDCl₃) :** *δ* 7.37 - 7,23 (m, 5H, CH-ar), 7.13 (bs, 1H, N*H*CH₃), 6.22 (d, *J* = 3.9 Hz, 1H, ON*H*R), 4.62 (dd, *J* = 6.6 Hz, *J* = 4.8 Hz, 1H, H-4), 3.66 (bs 1H, H-6), 3.62 (t, J = 6.7 Hz, 1H, H-3), 3.48 (d, *J* = 6.1 Hz, 2H, NC*H*₂Ph), 3.07 (d, *J* = 10.9 Hz, 1H, H-5), 3.00 (d, *J* = 9.9 Hz, 1H, H-2), 2.79 (d, *J =* 5.0 Hz, 3H, NC*H*₃), 2.37 (dd, *J* = 9.9 Hz, *J* = 6.7 Hz, 1H, H-2'), 2.21 (d, *J* = 10.9 Hz, *J* = 4.9 Hz, 1H, H-5') ppm. **RMN ¹³C (100 MHz, CDCl₃)** : *δ* 171.2 (C=O), 138.4 (C^{IV}-ar), 128.6 (CH-ar), 128.5 (CH-ar), 127.4 (CH-ar), 83.7 (C-4), 71.0 (C-6), 61.8 (C-5), 60.8 (C-2), 59.9 (N*C*H₂Ph), 50.2 (C-3), 26.0 (N*C*H₃) ppm. **HR-ESI-QToF MS** (mode positif): *mlz* calc pour C₁₄H₂₀N₃O₂ [M + H]⁺ 262.1550, trouvé 262.1553.

### Acétolyse directe du composé 3 en hétérobicycle 5:

L'acétolyse du composé **3** est réalisée de manière proche de la conversion de **3** en **4,** par addition de 0,8 mL d'acide sulfurique concentré (H₂SO₄ à 95 %) à un mélange réactionnel contenant le composé **3** (542 mg, 1.36 mmol), de l'acide acétique (7,2 mL) et de 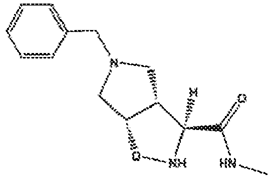 l'anhydride acétique (7,2 mL), puis chauffage du milieu à 46 °C pendant 6h30 sous argon. Une fois revenu à température ambiante, le mélange réactionnel est refroidi avec un bain de glace à 0 °C, puis les espèces acides sont neutralisées par une solution de soude 5M (ajout goutte à goutte sur 2h30). Lorsque le pH est d'environ 8, le milieu est versé dans une solution saturée de bicarbonate de sodium NaHCO₃ (700 mL) puis une extraction liquide-liquide (dichlorométhane, 5 x 100 mL) est réalisée. Après concentration et séchage, le produit brut qui contient des traces de composé *N*-acétylé **4** est purifié par flash chromatographie sur silice, pour donner **5** (320 mg, 90%).

Débenzylation et ouverture réductrice du lien N-O de **5** suivies d'hydrolyse basique : obtention du composé **1.1 :** Acide 3-pyrrolidine acétique, α-amino, 4-hydroxy (α*S,*3*R,*4*S*) ou acide α-amino-(4-hydroxy-pyrrolidin-3-yl)acétique (α*S*,3*R*,4*S*) On utilise un ballon de 100 mL, qui est rempli d'argon après y avoir introduit le composé **5** (112 mg, 0,43 mmol), On y ajoute de l'éthanol (10 mL) et du dichlorométhane (6 mL), puis 45 mg d'hydroxyde de palladium hydraté Pd(OH)₂/C à 20% (45 mg) sous argon. Le ballon est rempli d'hydrogène (1 atm) par 5 cycles de vide et admission d'hydrogène. Après 3 h d'agitation, 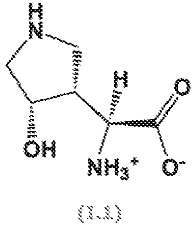 on constate (CCM) que la réaction est partielle, On ajoute 100 µL d'acide acétique AcOH *ca* 4 éq, et on agite encore 24 h sous hydrogène. On élimine le catalyseur par filtration sur Célite. Après concentration du brut réactionnel, le dérivé *N*-méthylamide intermédiaire est soumis à une hydrolyse basique en présence de lithine (180 mg, 4,3 mmol, 10 éq) dans un mélange THF : H₂O (1/1, v/v, 8 mL) pour 3 h. Après évaporation, le sel de lithium brut est soumis à chromatographie sur une colonne de silice C-18 préconditionnée (adaptée à un équipement automatisé Combiflash) éluée à l'eau, suivi d'un passage sur résine échangeuse d'ions (Dowex 50W X8), la résine étant, après dépôt du produit, lavée successivement par de l'eau, puis de l'eau ammoniacale (∼10 mL à 2,5 puis ∼10 mL à 5 %), L'évaporation à sec des fractions contenant le sel d'ammonium (CCM *n*-butanol/acide acétique/eau, 3/1/1 avec révélation par trempage dans une solution de ninhydrine dans !e *n*-butanol, puis chauffage) puis un passage sur une colonne C-18 (eau ultrapure) permet d'obtenir l'espèce zwitterionique **I.1** avec un rendement de 57 % sur 2 étapes (*50* % *pour* /*'énantiomère*). ***R_{f}*** = 0.18 (*n*-BuOH/H₂O/AcOH, 3/1/1). **[a]_{D}** = + 4.2 (c 0.5, DMSO/H₂O, 4/1). **RMN ¹H (400 MHz, D₂O) :** *δ* 4.58 (t, *J* = 3.0 Hz, 1H, H-4), 3.54 (d 1H, *J* = 9.5 Hz, H-6), 3.44 (dd, *J* = 8.6 Hz, *J* = 11.8 Hz, 1H, H-2), 3.39 - 336 (m, 2H, H-5, H-5'), 3.29 (d, *J* = 11.8 Hz, 1H, H-2'), 2.46 (ddd, *J* = 3.8 Hz, *J* = 9.1 Hz, *J* = 12.4 Hz, 1H, H-3) ppm. **RMN ¹³C (100 MHz, D₂O) :** *δ* 178.6 (C=O), 69.8 (C-4), 54.0 (C-6), 53.7 (C-5), 46.4 (C-3), 46.1 (C-2) ppm. **HR-ESI-QToF MS** (mode positif): *mlz* calc pour C₆H₁₃N₂O₃ [M + H]⁺ 161.0921, trouvé 161.0924.

**Le composé 1.2** (l'acide 3-pyrrolidine acétique, α-amino, 4-hydroxy (α*R*,3*S*,4*R*) ou acide α-amino-(4-hydroxy-pyrrollidin-3-yl)acétique (α*R*,3*S*,4*R*) est préparé selon une voie analogue à la précédente vers l'énantiomère **I.1.**

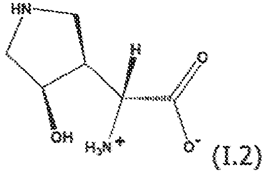

Elle est en théorie identique aux données pour I.1 sauf pour le pouvoir rotatoire [alpha] qui est de signe opposé ***R_{f}** =* 0.18 (*n*BuOH/H₂O/AcOH, 3/1/1). **[α]_{D}** = - 4.1 (*c*0.9, DMSO/H₂O, 4/1). **¹H NMR (400 MHz, D₂O) :** *δ* 4.58 (t, *J* = 2.9 Hz, 1H, H-4), 3.55 (d 1H, *J* = 9.5 Hz, H-6), 3.45 (dd, *J* = 8.7 Hz, *J* = 11.8 Hz, 1H, H-2), 3.39 - 3.36 (m, 2H, H-5, H-5'), 3.30 (d, *J* = 11.8 Hz, 1H, H-2'), 2.46 (ddd, *J* = 3.8 Hz, *J* = 9.0 Hz, *J* = 12.2 Hz, 1H, H-3) ppm. **¹³C NMR** (**100 MHz, D₂O) :** *δ* 178.4 (C=O), 69.8 (C-4), 53.9 (C-6), 53.7 (C-5), 46.3 (C-3), 46.1 (C-2) **ppm. ESI-QToF MS** (mode négatif): *m*/*z* [M *-* H]⁻ : 159.1, [2M - H]⁻ : 319.2 **HR-ESI-QToF MS** (mode positif): *m*/*z* calc pour C₆H₁₃N₂O₃ [M + H]⁺ 161.0921, trouvé 161.0923.

### PREPARATION DU COMPOSE I.3 : 3-Pyrrolidine N-méthylacétamide, α-acétamido, 4-hydroxy (αR,3S,4R) ou α-acétamido-(4-hydroxy-pyrrolidin-3-yl)-N-méthylacétamide (αR,3S,4R)

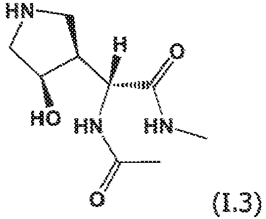

### Ouverture réductrice du lien N-O de l'énantiomère de 4 en présence d'hexacarbonyle de molybdène: obtention du composé 6

On introduit l'énantiomère de **4** (116 mg, 0,38 mmol) et l'hexacarbonyle de molybdène Mo(CO)₆ (111 mg, 0,42 mmol, 1,1 éq.) dans un ballon de 50 mL équipé d'un réfrigérant. Le montage est mis sous atmosphère inerte d'argon. On ajoute 6 mL d'acétonitrile 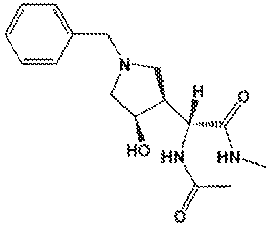 fraîchement distillé MeCN, puis, tout en agitant, 0,5 mL d'eau (qualité MilliQ) ; le mélange obtenu, chauffé au reflux (∼90°C) pendant 3 heures, passe du brun au noir. Après contrôle CCM (CH₂Cl₂/IPA, 8/2), le mélange réactionnel est concentré. La suspension obtenue est diluée dans le dichlorométhane CH₂Cl₂ (ou le tétrahydrofurane THF). Le liquide est filtré sur un lit de Célite (CH₂Cl_{2/}MeOH, 9/1 ou THF/MeOH 9/1) puis à nouveau concentré. Le mélange brut est purifié par chromatographie sur alumine basique Al₂O₃ (gradient d'élution CH₂Cl₂ puis CH₂Cl₂/MeOH, 8/2) pour donner 67 mg du composé **6** (57%). ***R_{f}* =** 0.18 (Ace/IPA, 1/1). **[a]_{D} = -** 31.4 (c0.6, DMSO). **RMN ¹H (400 MHz, CDCl₃):** *δ* 7.34 - 7.23 (m, 5H, CH-ar), 7.10 (d, *J* = 8.0 Hz, 1H, N*H*Ac), 6.86 (d, *J* = 4.4 Hz, 1H, N*H*CH₃), 4.53 (dd, *J* = 8.0 Hz, *J* = 5.5 Hz, 1H, H-6), 4.45 (td, *J* = 5.5 Hz, *J* = 2.8 Hz, 1H, H-4), 3.66 (d, *J* = 13.0 Hz, 1H, ½ NCH₂PH), 3.54 (d, *J* = 13.0 Hz, 1H, ½ NC*H*₂Ph), 2.91 (dd, *J* = 10.3 Hz, *J* = 5.6 Hz, 1H, H-5), 2.76 (d, *J* = 4.8 Hz, 3H, NC*H*₃), 2.64 - 2.48 (m, 4H, H-5', H-3, H-2, H-2'), 2.01 (s, 3H, NC(O)CH₃) ppm,

**HR-ESI-QToF MS** (mode positif): *m*/*z* calc pour C₁₆H₂₄N₃O₃ [M + H]⁺ 306.1812, trouvé 306.1811.

### Débenzylation de 6 en présence d'H₂ et de Pd(OH)₂/C: obtention du composé 1.3

Un ballon de 25 mL contenant du composé **6** (40 mg, 0.13 mmol) est soumis à 3 cycles vide/argon. On ajoute sous argon 5 ml d'éthanol EtOH et le mélange obtenu est soumis à 5 autres cycles vide/argon. On ajoute de l'oxyde de palladium à 20 % sur charbon Pd(OH)₂/C 20 % (14 mg, 0.024 mmol, 0.2 éq.). Le ballon est encore soumis à 5 cycles vide/argon et finalement à 5 cycles vide/hydrogène. Le mélange réactionnel 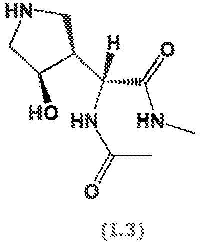 est agité pendant 1 heure sous hydrogène (1 atm) puis filtré sur un lit de Célite (lavages par CH₂Cl₂ et EtOH). Après concentration, le mélange brut est purifié par chromatographie sur une colonne de silice C-18 préconditionnée adaptée à un équipement automatisé (système Combiflash, élution à l'eau) pour donner 23 mg du composé **1.3** (82%). ***R_{f}** =* 0.31 (*n*-BuOH/H₂O/AcOH, 3/1/1). **[a]_{D}** = - 35.1 (*c* 0.6, DMSO). **RMN ¹H (400 MHz, D₂O) :** *δ* 4.42 (t, 1H, *J* = 4.0 Hz; H-4), 4.39 - 4.24 (m, 1H, H-6), 3.31 - 3.17 (m, 2H, H-2, H-5), 3.09 - 2.91 (m, 2H, H-2', H-5'), 2.77 (br s, 3H, NC*H*₃), 2.52 - 2.34 (m, 1H, H-3), 2.06 (br s, 3H, NC(O)CH₃) ppm.

**HR**-**ESI**-**QToF MS** (mode positif): *mlz* calc pour C₉H₁₅N-₃NaO₃ [M + Na]⁺ 236.1006, trouvé 236.1005.

### PREPARATION DU COMPOSE I.4 : 3-Pyrrolidine N-méthylacétamide, α-amino, 4-hydroxy (αS,3R,4S) ou α-amino-(4-hydroxy-pyrrollidin-3-yl)-N-méthylacétamide (aS,3R,4S)

Un ballon rond de 50 mL contenant le composé **5** (100 mg, 0.38 mmol) est mis sous vide, puis rempli d'argon (3 cycles). Toujours sous argon, 10 mL d'éthanol EtOH et 6 mL de dichlorométhane CH₂Cl₂ sont ajoutés et l'ensemble est mis sous pression réduite, puis sous argon (5 cycles). On 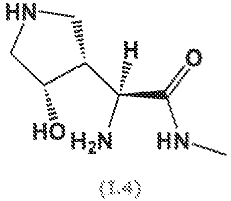 introduit 100 µL d'acide acétique AcOH et 40 mg d'hydroxyde de palladium sur charbon Pd(OH)₂/C 20 % (0,076 mmol, 0,2 éq.). Le ballon est finalement soumis à 5 cycles vide/argon puis à 5 cycles vide/hydrogène.

L'agitation sous hydrogène (1 atm) est maintenue une nuit, après quoi la CCM montre la conversion complète de **5.** Le catalyseur est séparé par filtration sur une couche de célite (EtOH/MeOH). Après concentration, the produit brut est traité par addition de méthanol MeOH (2 mL) et de toluène (5 mL), donnant le composé **1.4** sous forme d'un précipité pesant 36.7 mg, soit un rendement de 55 %.

Solide blanc; R*_{f}* = 0.19 (*n*-BuOH, CH₃CO₂H, H₂O, 3:1:1); [a]²²_{D} = +3.0 (*c* 0.5, H₂O).
**RMN ¹H (D₂O, 400 MHz**) δ 2.40 (m, 1H, H-3), 2.74 (s, 3H, *J_{gem}* = *J_{2,3}* =11.8 Hz, H-2), 3.23 (dd, 1H, *J_{2',3}* =8.5 Hz, *J_{gem}* =11.6 Hz, H-2'), 3.40 (m, 2H, H-5, H-5'), 3.50 (d, 1H, *J_{3,6} =* 9.9 Hz, H-6), 4.61 (m, 1H, *J_{3,4}* = 3.7 Hz, H-4); une COSY montre bien la corrélation H-6/H-3, puis H-3/H-2/H-2' et H-4;
**RMN ¹³C (D₂O, 100 MHz)** δ 26.07 (N*C*H₃), 45.37 (C-2), 47.29 (C-3), 53.07 (C-6), 53.70 (C-5), 69.31 (C-4), 175.9 (CO);
**MS: ESI** (mode positif): 174.0 [M+H]⁺, 347.0 [2M+H]⁺; **HRMS** Calc pour C₇H₁₅N₃O₂ Na₁, m/z = 196.1056; trouvé: 196.1051 (visible aussi : 174.1228 [M+H]⁺ ; 212.0788 [2M+H]⁺).

### PREPARATION DU COMPOSE RACEMIQUE 1.5 : Acide 3-pyrrolidine acétique, α-N-méthylamino, 4-hydroxy (3,4-cis) ou Acide α-N-méthylamino-(4-hydroxy-pyrrolidin-3-yl) acétique (3,4-cis)

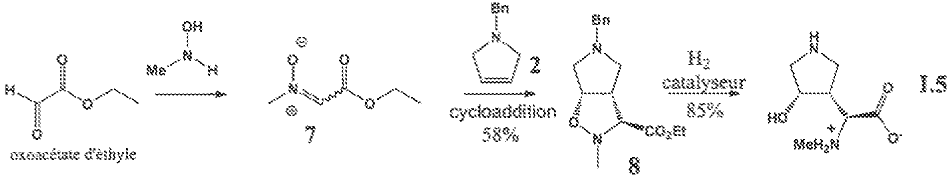

### Préparation de la nitrone 7 :

Elle est conforme à des protocoles déjà décrits [P. DeShong, C. M. Dicken, R. R. Staib, A. J. Freyer, S. W. Weinreb, J. Org. Chem. 1982, 47, 4397-4403 ; P. DeShong, J. M. Leginus, J. Org. Chem. 1984, 49, 3421-3423 ; Z.-Y. Hong, L. Liu, C.-C. Hsu, C.-H. Wong, Angew. Chem. Int. Ed. 2006, 45, 7417-7421].

L'oxoacétate d'éthyle (également nommé glyoxalate d'éthyle) (330 mg, 3,2 mmol) est dissous dans le toluène (7 mL), puis le chlorhydrate de *N*-méthylhydroxylamine (270 mg, 3,2 mmol) et NaHCO₃ (543 mg, 6,4 mmol) sont ajoutés et le mélange est agité pendant 24 h à température ambiante. Le mélange réactionnel est filtré et le solvant évaporé. La nitrone **7** (363 mg, 87%, en mélange *Z*/*E* 3:1) est utilisée directement dans l'étape suivante sans purification supplémentaire. **Aspect :** huile incolore. ***R_{f} =*** 0.5 (éther de pétrole / acétate d'éthyle 4:1). **RMN ¹H (300 MHz, CDCl₃) :** isomère *Z*: *δ* 7.23 (q, 1H, ⁴*J* = 0.7 Hz, C*H*), 4.24 (q, 2H, *J* = 7.1 Hz, O-C*H*₂-CH₃), 4.17 (d, 3H, ⁴*J* = 0.7 Hz, C*H*₃*-*N), 1.31 (t, 3H, *J* = 7.1 Hz, O-CH₂-C*H*₃) ppm ; isomère *E* : *δ* 7.11 (br s, 1H, C*H*)*,* 4.29 (q, 2H, *J* = 7.1 Hz, O-C*H*₂-CH₃), 5.32 (br s, 3H, C*H*₃-N), 1.32 (t, 3H, *J* = 7.1 Hz, OCH₂-C*H*₃) ppm.

### Cycloaddition de la nitrone 7 sur la benzyl-3-pyrroline 2 pour donner l'hétérobicycle 8 :

La nitrone **7** (300 mg, 2,29 mmol) et la benzyl-3-pyrroline **2** (0,8 mL, 4.20 mmol) sont dissoutes dans le toluène (3 mL) et agitées pendant 1 heure à 80°C sous irradiation micro-ondes. La CCM (AcOEt/ éther de pétrole 1/3) montre la transformation totale de la nitrone. Le solvant est ensuite évaporé avant la purification du cycloadduit **8** (390 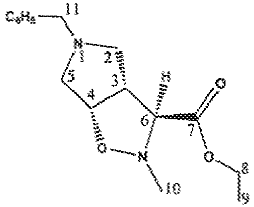 mg, 58%) sur colonne de silice (éluant : AcOEt/toluène 1/4) qui élimine des composés plus polaires également formés. **Aspect :** huile incolore. ***R_{f}*** = 0.4 (EtOAc/toluène 1/4), **RMN ¹H (500 MHz, CDCl₃)** : *δ* 735-7.2 (m, 5H, H-Ar), 4.60 (dd, 1H, ³*J* = 4.9 Hz, *J* = 7.4 Hz, H-4), 4.20 (q, 2H, ³*J* = 7.1 Hz, H-8, H-8'), 3.7 (d, 1H, ²*J* = 13.2 Hz, H-11), 3.58 (d, 1H, ²*J* = 13.2 Hz, H-11'), 3.24 (q, 1H, ³*J* = 7.1 Hz, H-3), 3.16 (br s, 1H, H-6), 3.00 (d, 1H, ³*J* = 11 Hz, H-5), 2.94 (d, 1H, ³*J* = 9.8 Hz, H-2), 2.78 (s, 3H, NC*H*₃), 2.28 (br s, 1H, H-2'), 2.19 (br s, 1H, H-5'), 1.27 (t, 3H, ³*J* = 7.1 Hz, H-9) ppm. **RMN ¹³C (126 MHz, CDCl₃):** *δ* 170.03 (C-7), 138.58, 128.73, 128.41, 127.17 (C-Ar), 81.03 (C-4), 75.48 (C-6), 61.26 (C-8), 59.08 (C-11), 58.77 (C-5), 56.79 (C-2), 52.54 (C-3), 43.85 (N*C*H₃), 14.24 (C-9) ppm. **HRMS ESI :** *mlz* calc pour C₁₆H₂₃N₂O₃ [M+H]⁺ : 291.1703; trouvé: 291.1702.

### Réduction du bicycle 8 en acide α-N-méthylamino-(4-hydroxy-pyrrollidin-3-yl) acétique, 3,4-cis I.5 :

Le cyloadduit **8** (150 mg, 517 µmol) est dissous dans un mélange THF/eau 1:1 (15 mL). Le catalyseur Pd(OH)₂/C 20% (20 mg) est ajouté, puis le mélange réactionnel est agité sous atmosphère de H₂ à température ambiante. Ces conditions entraînent la débenzylation et l'ouverture de la liaison N-O ainsi que la saponification de l'ester, qui sont totales après 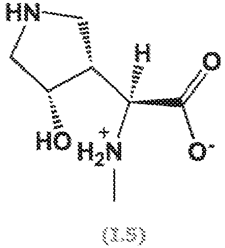 ∼12 h sous agitation. Le mélange réactionnel est filtré sur célite puis le solvant est évaporé. Une purification sur colonne en phase inverse suivie d'une recristallisation dans le MeOH permet d'obtenir le composé **1.5** (80 mg, 85%). **Aspect :** cristaux blancs ; pf 192°C (MeOH). **RMN ¹H (400 MHz, D₂O) :** δ 4.54 (t, 1H, ³*J* = 3.2 Hz, H-4), 3.36 (dd, 1H, ³*J* = 3.3 Hz, ²*J* = 12.8 Hz, H-5), 3.31 (dd, 1H, ³*J* = 0.9 Hz, ²*J* = 12.7 Hz, H-5'), 3.26 (dd, 1H, ³*J =* 8.5 Hz, ²*J* = 11.6 Hz, H-2), 3.17 (t, 1H, ²*J* = ³*J* = 11.7 Hz, H-2'), 3.17 (d, 1H, *J* = 10 Hz, H-6), 2.34 (s, 3H, NC*H*₃), 2.32 (m, 1H, H-3) ppm. **RMN ¹³C (101 MHz, D₂O):** δ 178.99 (C-7), 69.41 (C-4), 62.78 (C-6), 53.87 (C-5), 46.04 (C-3), 45.79 (C-2), 33.47 (N*C*H₃) ppm, **HRMS CI :** *mlz* calc pour C₇H₁₅N₂O₃ [M+H]⁺ : 175.1078; trouvé : 175.1077.

### PREPARATION DU COMPOSE RACEMIQUE 1.6: 3-(1,2-Diaminoéthyl)-4-hydroxy-pyrrolidine (3,4-cis)

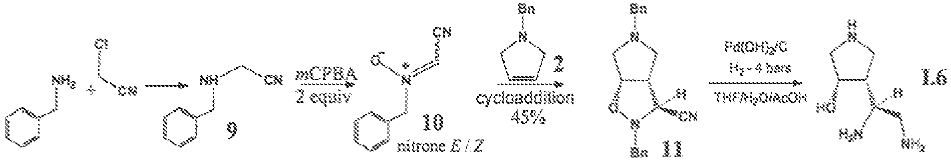

La nitrone a été préparée à partir de la *N*-cyanométhylbenzylamine **9** comme décrit par H. Tokuyama, T. Kuboyama, A. Amano, T. Yamashita, T. Fukuyama, *Synthesis* **2000,** 1299-1304.

### Obtention de la N-cyanométhylbenzylamine 9:

A une solution de benzylamine (1 mL, 9,15 mmol) dans l'acétonitrile (50 mL), on ajoute le chloroacétonitrile (870 µL, 13,7 mmol) et K₂CO₃ (2,53 g, 18,3 mmol) sous atmosphère d'argon. Après 15 h d'agitation à 60 °C, le mélange est filtré sur célite puis le filtrat est concentré. La *N*-cyanométhylbenzyiamine **9** (1.03 g, 77%) est purifiée par colonne chromatographique sur gel de silice (éluant : éther de pétrole / acétate d'éthyle 3:1).

**Aspect** : huile incolore. ***R_{f}*** = 0.25 (éther de pétrole / acétate d'éthyle 2:1). **RMN ¹H (300 MHz, CDCl₃) :** *δ* 7.4-7.25 (m, 5H, H-Ar), 3.94 (s, 2H, C*H*₂-CN), 3.57 (s, 2H, C*H*₂-Ph) ppm. **RMN ¹³C (75 MHz,** CDCl₃) : *δ* 138.0, 128.5, 128.3, 127.5, 117.8, 52.2, 36.1 ppm.

### Oxydation de la N-cyanométhylbenzylamine 9 en cyanonitrone 10:

La *N*-cyanométhylbenzylamine **9** (530 mg, 3,62 mmol) est dissoute dans le dichlorométhane anhydre (20 mL), puis l'acide méta-chloroperbenzoïque (*m*-CPBA, 2 g, 8 mmol) est ajouté par petites portions à 0 °C. Après 30 min d'agitation à 0 °C, puis 1 h à température ambiante, la réaction est stoppée par ajout d'une solution de Na₂S₂O₃ 10% (10 mL), puis d'une solution saturée de 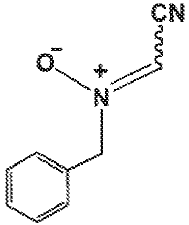 K₂CO₃ (10 mL). Le mélange est agité pendant encore 30 min avant d'être extrait trois fois au dichlorométhane. Les phases organiques sont lavées avec de la saumure et séchées avec MgSO₄. Une purification sur silice (éluant : éther de pétrole / acétate d'éthyle 4:1) permet d'obtenir la cyanonitrone **10** (550 mg, 95%) sous deux formes isomères Zet *E* (rapport *Z*/*E* 3:1). **Aspect :** cristaux blancs. ***R_{f}*** (*E*) *=* 0.7 (éther de pétrole / acétate d'éthyle 3:1) ; ***R_{f}*** (*Z*) = 0.4 (éther de pétrole / acétate d'éthyie 3:1). **RMN ¹H (300 MHz**, **CDCl₃) :** *δ* 7.6-7.3 (m, 5H, H-Ar), 6.67 (s, 1H, C*H*-CN *E*), 6.57 (s, 1H, C*H-*CN *Z*), 5.32 (s, 2H, Ph-C*H*₂ *E*), 5.01 (s, 2H, Ph-C*H*₂ *Z*) ppm. **RMN ¹³C (75 MHz, CDCl₃) :** *δ* 132-129 (C-Ar), 113.2 (CN, *E*), 112.4 (CN, *Z*), 107.5 (CH, *E* and *Z*), 71.7 (*C*H₂, *Z*), 70.1 (*C*H₂, *E*) ppm.

### Cycloaddition de la cyanonitrone 10 sur 2 pour donner l'hétérobicycle 11:

La cyanonitrone **10** (550 mg, 3,44 mmol) et la benzyl-3-pyrroline **2** (1.3 mL, 6,83 mmol) sont dissoutes dans le toluène (4 mL) et agitées pendant 2 h à 80°C sous irradiation micro-ondes (température affichée : 80°C). Le solvant est ensuite évaporé avant purification et séparation sur colonne de silice (éludant : AcOEt/toluène 1/10) d'un 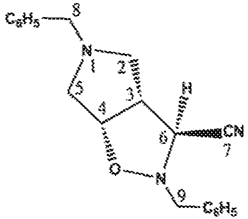 régioisomère (60 mg, 6%) pour donner le cycloadduit racémique attendu **11** (500 mg, 45%). **Aspect :** huile jaune. ***R_{f}*** = 0.4 (EtOAc/toluène 1/4). **RMN ¹H (400 MHz, 60°C, Toluene-D8) :** *δ*7.3-7.0 (m, 10H), 4.28 (ddd, 1H, *J* = 3.4 Hz, *J* = 6.0 Hz, *J* = 7.5 Hz, H-4), 4.17 (d, 1H, *J* = 13.4 Hz, H-9), 3.81 (d, 1H, *J* = 13.4 Hz, H-9'), 3.30 (d, 1H, *J* = 13.1 Hz, H-8), 3.23 (d, 1H, *J* = 13.1 Hz, H-8'), 3.06 (d, 1H, *J* = 3.6 Hz, H-6), 2.79 (ddd, 1H, *J* = 3,8 Hz, *J* = 7.9 Hz, *J* = 11.6 Hz, H-3), 2.47 (ddd, 1H, *J* = 3.0 Hz, *J* = 10.0 Hz, H-5), 2.24 (br m, 1H, H-5'), 2.16 (br m, 1H, H-2), 2.07 (br m, 1H, H-2') ppm. **RMN ¹³C (100 MHz, 60°C, Toluene-D₈**) : *δ* 139.3, 136.7, 129.7, 129.7, 129.0, 129.0, 128.8, 128.8,128.8, 128.8, 128.1, 127.6, 116.5 (C-7), 81.4 C-4), 59.5 (C-8), 59.3 (3C, C-5, C-6, C-9), 57.0 (C-2), 53.0 (C-3) ppm. **HRMS :** *m*/*z* calc pour C₂₀H₂₂N₃O [M+H]⁺ : 320.1757; trouvé: 320.1767.

### Réduction du bicycle 11 en 3-(1,2-diaminoéthyl)-4-hydroxy-pyrrolidine 3,4-cis, I.6 :

Le cycloadduit **11** (40 mg, 125 µmol) est dissous dans un mélange THF/eau/AcOH 10:10:1 (3 mL). Le catalyseur Pd(OH)_{2/}C 20% (10 mg) est ajouté au milieu, puis le mélange est agité sous 4 bars de H₂ à température ambiante pendant environ 40 h. Un test par CCM (éluant *n*BuOH-eau-AcOH 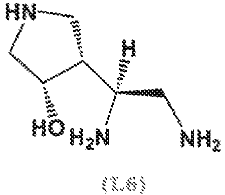 3:1:1) montre la conversion totale du produit de départ. Le milieu est filtré sur célite puis le solvant est évaporé (23 mg). Une analyse par spectrométrie de masse montre un ion très majoritaire correspondant à la polyamine attendue **I.6** et un autre pour un produit mineur encore benzylé. La RMN ¹³C confirme l'existence de ces 2 produits dans une proportion de 1:3 à 1:4. **RMN ¹³C (75 MHz, D₂O)** : *δ* 69.20 (C-4), 53.73 (C-5), 48.14 (C-6), 45.72 (C-3), 45.09 (C-2), 42.52 (C-7) ppm. **HRMS (ESI,** mode positif; solvant d'infusion: eau/MeOH 1:1) : *m*/*z* calc pour C₆H₁₆N₃O [M+H]⁺ 146.1288; trouvé: 146.1281.

### II. Activités biologiques

### II.1 Evaluation de l'activité modutatrice des taux de glutamate et d'aspartate extracellulaires

L'une des approches méthodologiques répandues, tant dans les laboratoires académiques que dans les laboratoires industriels, et utilisées afin de suivre les concentrations extracellulaires de glutamate (Glu) et d'aspartate (Asp) avant, pendant et après l'administration d'agents pharmacologiques ciblant leurs récepteurs est la microdialyse Intracérébrale **[7].** Ce type de test a donc été employé, afin de suivre des effets *in vivo* des molécules selon l'invention en comparaison avec ceux de l'acide kainique dont les molécules synthétisées sont des analogues structuraux (pyrrolidines substituées),

### Sondes de microdialyse

Des sondes de microdialyse concentriques avec une membrane en cellulose régénérée (Spectra/Por hollow fiber; arrêt moléculaire 6000 Da, 225 µm de diamètre externe, longueur de 3 mm, Spectrum Medical Industries, Los Angeles, CA, USA) et des tubulures en silice fondue (40 µm i.d×105 µm o.d., Polymicro Technology, Phoenix, AZ, USA) ont été fabriquées selon un design déjà publié (Bert et al., 1996, **[26]).** Elles ont été perfusées à un débit de 1µL/min avec un liquide céphalo-rachidien artificiel ou LCR (145,0 mM NaCl ; 2,7 mM KCl ; 1,0 mM MgCl₂ ; 1,2 mM CaCl₂ ; 0,45 mM NaH₂PO₄ ; 1,55 mM Na₂HPO₄ ; pH 7,4) à l'aide une pompe Harvard (Model 22, South Natick, MA, USA).

### Implantation des sondes de microdialyse

Les expériences de microdialyse *in vivo* ont été menées sur des rats mâles de souche Wistar (pesant en moyenne environ 300 g, fournisseur : Charles River, L'Arbresle, France) anesthésiés avec de l'uréthane (dose de 1,4 g/kg par voie i.p., fournisseur : Sigma-Aldrich, St. Louis, MO, USA). Les sondes ont été implantées dans le striatum dorso-latéral selon les coordonnées stéréotaxiques suivantes relatives au bregma : antéro-postériorité 0 mm, latéralité +3,5 mm, profondeur +6,5 mm sous la surface corticale. Les dialysats (échantillons) ont été recueillis à partir du début de la 4^{e} heure suivant l'implantation de la sonde. Les échantillons ont été prélevés toutes les 5 minutes dans des tubes PCR de 200 µL (Axygene, Union City, CA, USA) et immédiatement congelés à -20 °C. Les échantillons ont été analysés ultérieurement, afin de déterminer le contenu en glutamate et en aspartate. Les agents pharmacologiques et les composés selon l'invention ont été administrés *in situ* par microdialyse inverse en changeant la perfusion en entrée de sonde du LCR sans agent pharmacologique à celui contenant le ou les agents pharmacologiques (Bert et al., 2002 **[27];** Parrot et al., 2003 **[28]).**

### Electrophorèse capillaire (EC)

Les analyses par EC ont été effectuées avec un système P/ACE MDQ (Beckman-Coulter, Fullerton, CA, USA) équipé d'un détecteur externe LIF ZETALIF (Picometrics, Toulouse, France). La détection a été effectuée par fluorescence à la longueur d'onde de 442 nm et générée par un laser He-Cd Omnichrome (Chino, CA, USA).

L'intensité d'émission a été recueillie à 490 nm. Avant l'analyse, les échantillons ont été dérivés au naphthalène-23-dicarboxaldéhyde avec des ions cyanure comme agent nucléophile (pH 8,7 ; tampon borate 500 mM). Les séparations ont été menées dans un capillaire en silice fondue de 50 cm de diamètre interne et 375 µm de diamètre externe de 63 cm de long avec une longueur utile de 52 cm (Polymicro Technology) en utilisant le protocole entièrement détaillé et validé précédemment (Sauvinet et al., 2003 **[29];** Bert et al., 2002 **[27]).** Les concentrations en Glu et en Asp mesurées dans les échantillons striataux étaient au-dessus des limites de détection et ont été déterminées par interpolation grâce à des gammes étalon.

### Réactifs

Tous les réactifs de l'étude neurochimique (à l'exception des composés selon l'invention) ont été achetés chez Sigma-Aldrich (St Louis, MO, USA). Les solutions mères des molécules synthétisées, du kainate (KA, agoniste des récepteurs KA au glutamate), du CNQX, du 6-cyano-7-nitroquinoxaline-2,3(1H,4H)-dione (CAS-No. 115066-14-3, antagoniste des récepteurs KA), du Glu et de l'Asp ont été aliquotées à 1 ou 0,1 mM et stockées à -30 °C. Avant leur administration, les agents pharmacologiques testés ont été dilués dans du LCR artificiel.

### Analyses des données

Les données finales ont été exprimées en % de la moyenne des quatre valeurs précédant l'administration du KA.

### Résultats et discussion

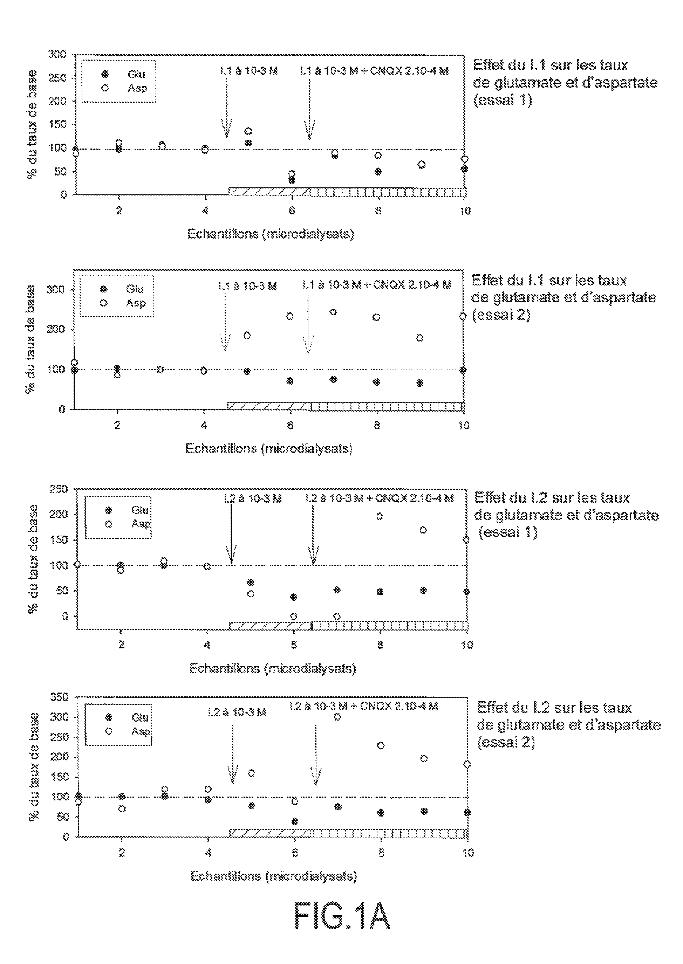

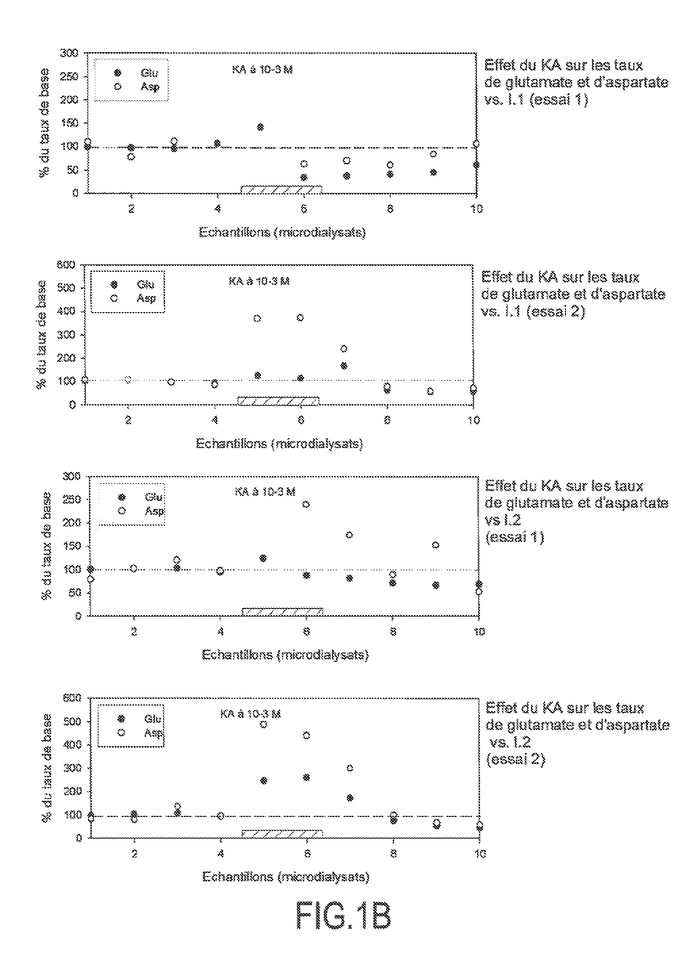

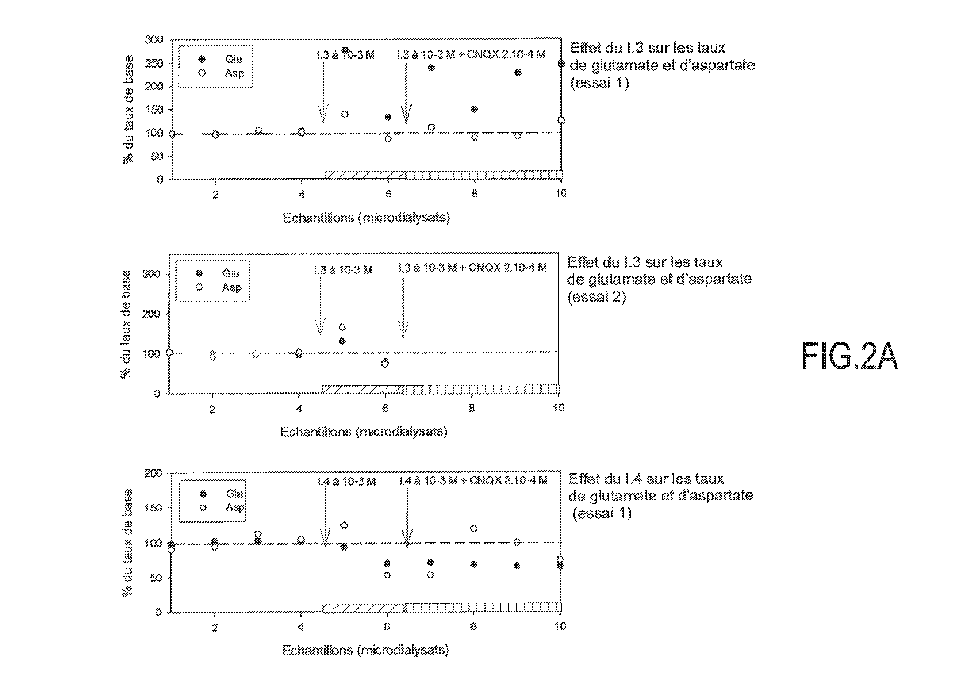

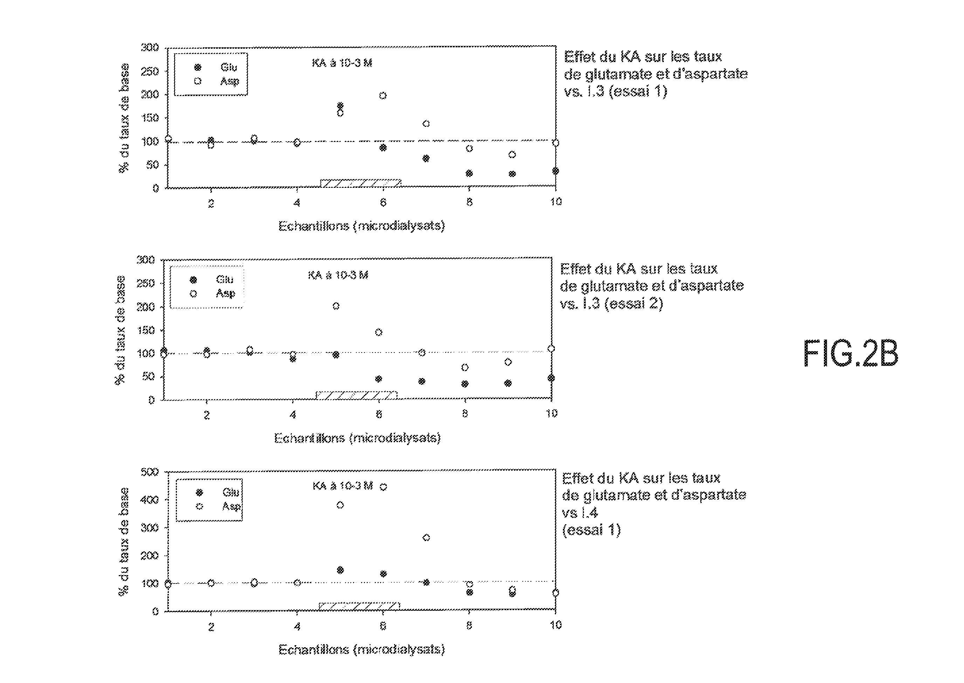

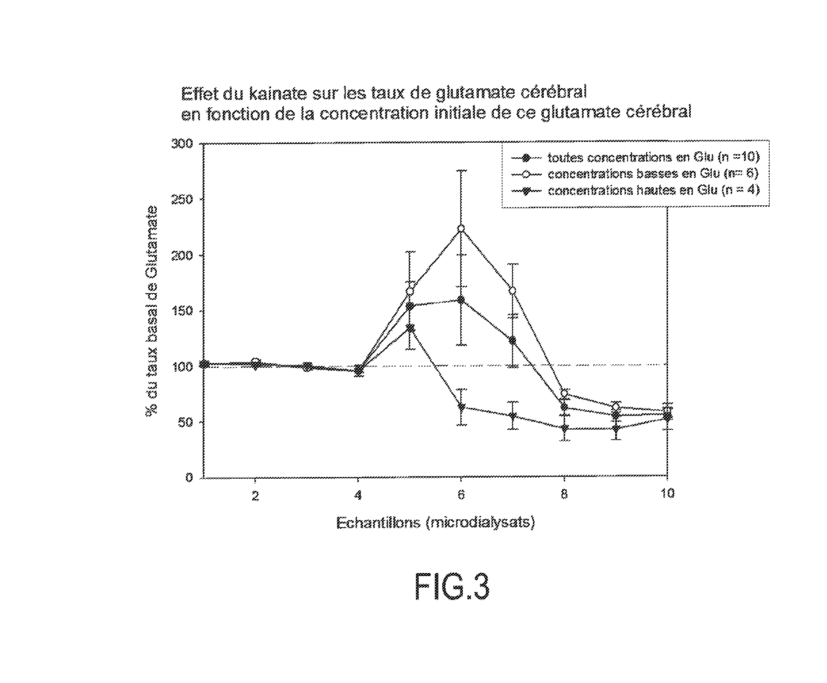

Quatre molécules selon l'invention ont été testées *in vivo* sur le cerveau (structure cérébrale : striatum) d'animaux entiers anesthésiés. Leurs effets ont été comparés à celui du kainate (KA), agoniste des récepteurs de type KA au glutamate. Les résultats sont présentés sur les **Figures 1A****,** **1B****,** **2A****,** **2B** **et** **3** annexées.

Les résultats obtenus montrent que la réponse au KA a été variable d'un sujet à l'autre. Les taux de Glutamate et d'Aspartate ont été affectés par l'administration de KA à 1 mmol/L. Ce contrôle systématique nous permet ainsi de vérifier la réactivité des neurones striataux vis-à-vis des récepteurs au KA. Les **Figures 1A** **et** **1B** comparent les effets des molécules **I.1** et **I.2** avec et sans kainate (KA) respectivement, les **Figures 2A** et **2B** comparent les effets des **I.3** et **I.4** avec et sans kainate (KA) respectivement, et la **Figure 3** montre les effets du kainate (KA) en fonction de la concentration de Glutamate endogène de départ.
Composé **I.1** à 1 mmol/L (n=2) : Le composé **I.1** induit des effets similaires au KA qui pourraient être atténués puisque les taux de Glu et d'Asp tendent à revenir vers les taux basaux.
Composé **I.2** à 1 mmol/L (n=2) : Le composé **I.2** induit une diminution des taux de Glu et d'Asp (effet similaire à ceux du kainate), et le CNQX semble en partie atténuer ses effets de manière significative puisque les taux d'Asp sont augmentés suite à sa co-administration avec le composé **I.2.**
Composé **I.3** à 1 mmol/L (n=2) : Le composé **I.3** induit un effet similaire à ceux du kainate, et le CNQX semble en partie atténuer ses effets de manière significative puisque les taux d'Asp sont augmentés suite à sa co-administration avec le composé **I**.**3** (n=1).
Composé **I.4** à 1 mmol/L (n=1) : Le composé **I.4** induit une diminution des taux de Glu et d'Asp (effet similaire à ceux du kainate), et le CNQX semble en partie atténuer ses effets de manière significative puisque les taux d'Asp sont augmentés suite à sa co-administration avec le composé **I.4 (n= 1).**

**Tableau 1 synthétique des résultants préliminaires**

| | Composé **I.1** | Composé **I.2** | Composé **I.3** | Composé **I.4** |
|---|---|---|---|---|
| Effet sur Glu et/ou Asp | oui | oui | oui | oui |
| Action par les récepteurs KA | non déterminée | oui pour Asp | non déterminée | oui pour Asp |
| Potentialité pharmacologique pour moduler le système Glu | oui | oui | oui | oui |

Les résultats montrent que les composés selon l'invention, qui sont des analogues structuraux de l'acide kainique, agissent sur les taux de glutamate et d'aspartate extracellulaires.

Les composés selon l'invention, et en particulier, les composés **I.1, I.2, I.3** et **I.4** présentent des actions inhibitrices sur le glutamate quand les taux de glutamate sont élevés et sont, de ce faît, de bons candidats pour le traitement, et en particulier pour le traitement des êtres humains, de pathologies comme la maladie de Parkinson, l'épilepsie, les troubles addictifs ou l'ischémie **[2-5; 7].**

### II.2 Détermination des récepteurs cibles

Afin de déterminer les récepteurs cibles des composés selon l'invention, des expériences d'inhibition de liaison spécifique ont été réalisées *in vitro.* Pharmacologie in vitre : tests de liaison spécifiques
Soixante récepteurs ont été testés dans l'étude selon des méthodes standardisées en utilisant leur radioligand spécifique comme précédemment rapporté **[30-88].**

Les résultats sont exprimables en % de la liaison contrôle spécifique [(liaison spécifique mesurée/liaison spécifique contrôle)×100] et comme % d'inhibition de la liaison contrôle spécifique [100 -(liaison spécifique mesurée/liaison spécifique contrôle) x100] obtenue en présence de l'un des deux composés testés (**I.2** et **I.4).** Ces expériences (**Tableau 2** ci-après) ont révélé que les composés **I.2** et **I.4** modifient la liaison spécifique d'un agoniste contrôle des récepteurs de type 1 aux endocannabinoïdes (C8-1) (selon le test décrit dans la référence **[83])**.

**Tableau 2: Valeurs du % d'inhibition de la liaison spécifique contrôle à 10 µM (n=2) pour les composés testés**

| % d'inhibition de la liaison spécifique contrôle à 10 µM | Composé I.2 | composé I.4 |
|---|---|---|
| CB1 vis-à-vis de son ligand agoniste | +29 % | +29% |

Les % d'inhibition correspondent à un effet significatif d'après une analyse sur 60 récepteurs cibles (n=2) avec une représentation de type 'box and whisker' réalisée pour chaque composé.

Ces résultats, ainsi que les résultats neurochimiques, qui montrent que les composés **I.2** et **I.4** entraînent une diminution des taux de glutamate extracellulaire cérébral, sont cohérents avec la littérature neuropharmacologique. En effet, un agoniste des récepteurs CB-1, comme le WIN 55,212-2, induit également une diminution des taux de glutamate dans le striatum de préparations ex vivo et in vivo **[89-90],** alors que les antagonistes CB-1, comme le rimonabant ou le SR-141716A, font augmenter ou n'affectent pas les taux de glutamate extracellulaire **[91-92]**. Les composés **I.2** et **I.4** induisent des effets proches de ceux observés avec des agents cannabinoidergiques.

Les récepteurs CB-1 sont impliqués dans l'addiction **[93]** et la schizophrénie **[94],** la dépression et la douleur **[95],** les maladies neurodégénératives **[96],** mais également dans le contrôle de la prise alimentaire **[97]** et des fonctions cardiaques et digestives **[98].** Par conséquent, les composés selon l'invention, et en particulier les composés **I.2** et **I.4,** sont de bons candidats pour le traitement de telles pathologies.

### II.3 Passage de la barrière hématoencéphalique

Une étude a été réalisée par la plate-forme BIP (Centre de Recherche en Neurosciences de Lyon, Lyon, France) pour comparer l'influx de quatre composés d'intérêt au travers de la barrière sang / liquide céphalo-rachidien. L'étude a été réalisée à l'aide d'un modèle *in vitro* de l'épithélium choroïdien reconstituant cette interface. Les cellules choroïdiennes ont été exposées par leur pôle basolatéral aux composés à la concentration de 150 µM, pendant 60 minutes. La perméabilité des monocouches cellulaires au saccharose a été analysée au terme de ces transferts, pour déceler une éventuelle toxicité des composés.

### METHODES

### Culture primaire de cellules épithéliales choroïdiennes

Les cellules épithéliales sont isolées à partir de plexus choroïdes de ventricule latéral prélevés sur des rats nouveau-nés comme précédemment décrit **[99-100].** Les cellules sont ensemencées sur supports microporeux (Transwell PC, 0,4 µm de taille de pores, 0,33 cm² surface, 6,5 mm diamètre), et cultivées en DMEM - F12 (1:1) supplémenté par 10% FCS, 2 mM glutamine et 50 µg/ml gentamycine, ainsi que divers facteurs de croissance **[99].** Le milieu est renouvelé tous les deux jours jusqu'au jour d'expérimentation.

### Mesure de perméabilité

Les mesures de transfert sont réalisées en cinétique (3 temps), sous agitation (200 rpm), à 37°C, dans du tampon Ringer-Hepes (RH). Les volumes utilisés dans les deux compartiments lors du transfert sont ajustés de façon à minimiser tout effet de pression hydrostatique. Les inserts sont rincés en RH avant d'initier l'étude de perméabilité. Pour chaque composé, le transfert est réalisé en parallèle sur trois inserts laminine (sans cellule) et trois inserts avec monocouche cellulaire.

### Influx des composés I.1, I.2, I.3 et I.4

Des mesures d'influx (du sang vers le liquide céphalo-rachidien) ont été réalisées au cours d'une étude cinétique **[101]** (périodes de 20 min) pour les quatre composés d'intérêt. Brièvement, le composé d'intérêt, dilué à 150 µM en RH est placé dans le compartiment inférieur d'une plaque de culture multi puits, qui reproduit le compartiment sanguin. Le transfert est initié en plaçant un insert de culture contenant du RH dans le compartiment supérieur, lequel correspond au liquide céphalo-rachidien. A intervalles réguliers, un aliquote du milieu supérieur est prélevé et remplacé par un volume équivalent de RH préchauffé à 37°C. A l'issue du transfert, le milieu inférieur est également prélevé pour dosage du composé résiduel. Les échantillons prélevés sont analysés par chromatographie liquide à haute performance (CLHP) sur chaîne LC10, Shimadzu system (Duisburg, Germany), équipée d'un détecteur spectrophotométrique.

### Perméabilité au saccharose

A l'issue du transfert des composés d'intérêt, le [¹⁴C]-saccharose est ajouté sous forme d'un aliquote dans le compartiment supérieur des inserts. A intervalles réguliers, les inserts sont transférés dans des puits voisins contenant du RH préchauffé à 37°C. Les milieux prélevés dans les puits et dans les inserts à l'issue du transfert de saccharose sont analysés par comptage en scintillation liquide dans un compteur β Canberra Packard TRI-CARB 1600TR.

### Calcul des coefficients de perméabilité

La méthode de calcul est décrite en détail dans **[101].** Brièvement, les prélèvements successifs du compartiment accepteur au cours des cinétiques de transfert permettent de tracer des courbes de clairance. La pente de ces courbes représentant la vitesse de clairance peut être assimilée au produit perméabilité x surface du composé (PS en µl.min⁻¹ per filter).

Le coefficient de perméabilité de la monocouche cellulaire Pe (en cm.min⁻¹) est calculé à partir des PS déterminés sur filtres laminine et filtres avec cellules et en tenant compte de la surface d'échange.

### Méthode de dosage des composés I.1 et I.2

Une technique de dosage par CLHP précédée d'une étape de dérivation des amines primaires a été mise en oeuvre à partir d'une méthode existante **[102]** pour les deux composés **I.1** et **I.2.** La réaction de dérivation s'effectue avec de l'OPA (o-phthaldialdéhyde, 10 mg), 200 µl de méthanol, 1,8 ml de tampon borate 200 mM à pH 9,5 et 10 µl de 2-β-mercaptoéthanol. La réaction volume à volume d'échantillon s'effectue pendant 2 min à température ambiante. Le dérivé est stable 30 à 40 min. Conditions analytiques : Colonne RP-18, 15 cm, 5 µm. Phase mobile : A : 90/10 (tampon phosphate de potassium 50mM pH6 / méthanol) ; B : 100% méthanol. Détection: 340 nm. Débit : 1 ml/min. Volume d'injection : 20 µl. Gradient utilisé min (A:B) : 0-5 (83 :17) ; 6-11 (35 :65) ; 12-17 (83 :17).

### Méthode de dosage des composés I.3 et I.4

Une technique de dosage par CLHP précédée d'une étape de dérivation des amines secondaires au Fmoc (chlorure de fluorenylméthyloxycarbonyl ; à 5 g/l dans l'acétonitrile a été développée pour les deux composés **I.3** et **I.4.** La réaction de dérivation a été réalisée pendant 30 min à température ambiante dans du tampon borate 200mM à pH 9,5 (25 µl d'échantillon + 20 µl de tampon borate + 5 µl de la solution à 5g/L de Fmoc). Le dérivé formé est stable pendant 2 h.

Conditions analytiques: Colonne RP-18, 15 cm, 5µm. Phase mobile : A : 90/10 (tampon acétate de sodium 50 mM pH 4,2 / acétonitrile) ; B : 100% acétonitrile. Détection: 230 nm. Débit : 1 ml/min. Volume d'injection ; 20 µl. Gradient utilisé pour la molécule **I.3** min (A:B) : 0 (75:25) ; 8,5 (67:33) ; 9,5-20 (20:80) ; 21-26 (75:25). Gradient utilisé pour la molécule **I.4** min (A:B) : 0 (65:35) ; 18 (40:60) ; 19-29 (20:80) ; 30-35 (35:35)

Les expériences réalisées ont montré *in vitro* que les composés selon l'invention pouvaient passer la barrière hématoencéphalique.

Par ailleurs, étant donné un faible taux de pénétration (≤ 0.3 x 10⁻³ cm/min) du compartiment sanguin périphérique vers le compartiment cérébral pour les quatre composés testés (**I.1, I.2**, **I.3** et **I.4)**, les composés selon l'invention pourraient induire des effets périphériques avec peu d'effets secondaires centraux.

Ceci suggère fortement une possible utilisation pharmacologique de tels composés pour agir sur différents organes et tissues périphériques, dans le traitement des cancers **[103],** de l'obésité **[104],** des troubles squeletto-musculaires **[105],** des maladies cardio-vasculaires et des maladies des voies digestives **[98],** en plus des traitements précédemment cités.

### Références citées

1. Fonnum F, (1984). Glutamate: a neurotransmitter in mammalian brain. J Neurochem, 42, 1-11,
2. Farber NB, Newcomer JW, Olney JW, (1998). The glutamate synapse in neuropsychiatric disorders, Focus on schizophrenia and Alzheimer's disease. Prog Brain Res, 116, 421-437.
3. Avoli M, Louvel J, Pumain R, Kohling R, (2005). Cellular and molecular mechanisms of epilepsy in the human brain. Prog Neurobiol, 77,166-200.
4. Javitt DC, (2004). Glutamate as a therapeutic target in psychiatric disorders. Mol Psychiatry, 9,984-997, 979.
5. Gubellini P, Pisani A, Centonze D, Bernardi G, Calabresi P, (2004). Metabotropic glutamate receptors and striatal synaptic plasticity: implications for neurological diseases. Prog Neurobiol, 74, 271-300.
6. Bevan MD, Atherton JF, Baufreton J, (2006). Cellular principles underlying normal and pathological activity in the subthalamic nucleus. Curr Opin Neurobiol, 16, 621-628.
7. Parrot S, Renaud B, Zimmer L, Denoroy L, (2011) Monitoring neurotransmitter amino acids by microdialysis: pharmacodynamics applications. In:"Applications of Microdialysis in Pharmaceutical Science" (Ed: T-H Tsai). Wiley.
8. C. Berini, N. Pelloux-Léon, F. Minassian, J.-N, Denis Org. Biomol. Chem. 2009, 7, 4512-16
9. J. E. Baldwin, R. M. Adlington, D. W. Collins, C. J. Schofield Tetrahedron 1990, 46, 4733-4748
10. S. Horii, H. Fukase, E. Higashide, M. Yoneda, H. Nishida, H. Sakai, A. Hirota, I. Isogai J. Antibiot. 1985, 38, 302
11. Zanobini, A.; Gensini, M.; Magull, J.; Vidovic, D.; Kozhushkov, S. I.; Brandi, A.; de Meijere, A. Eur. J. Org. Chem. 2004, 4158-66
12. Thèse de J. A. Stanko, Duke University, USA, 2009
13. U. Chiacchio, A. Corsaro, D. Iannazzo, A. Piperno, V. Pistarà, A. Rescifina, R. Romeo, G. Sindona, G. Romeo, Tetrahedron Asymm. 2003,14,2717-2723.
14. L. Weselinski, E. Slyk, J. Jurczak Tetrahedron Lett. 2011, 25, 381-384.
15. U. Chiacchio, A. Corsaro, V. Pistarà, A. Rescifina, D. Iannazzo, A. Piperno, G. Romeo, R. Romeo, G. Grassi Eur. J. Org. Chem. 2002,1206-1212.
16. U. Chiacchio, F. Genovese, D. Iannazzo, V. Librando, P. Merino A. Rescifina, R. Romeo, A. Procopio, G. Romeo, Tetrahedron 2004, 60, 441-448.
17. D. Iannazzo, C. Carnovale, S. V. Giofrè, R. Ettari, G. Romeo, R. Romeo, G. Lanza, U. Chiacchio Synlett 2011, 245-248.
18. U. Chiacchio, A. Corsaro, G. Gumina, A. Rescifina, D. Iannazzo, A. Piperno, G. Romeo, R. Romeo 3. Org. Chem. 1999, 64,9321-9327.
19. K. Kasahara, H. Iida, C. Kibayashi J. Org. Chem. 1989, 54, 2225-2233.
20. V. Ondrus, M. Orsag, L. Fisera, N. Pronayova, Tetrahedron 1999, 55, 10425-10436.
21. N. Morita, K. Fukui, J. Irikuchi, H. Sato, Y. Takano, I. Okamoto, H. Ishibashi, O.Tamura J. Org. Chem. 2008, 73, 7164-7174
22. O.Tamura, N. Morita, Y. Takano, K. Fukui, I. Okamoto, X. Huang, Y. Tsutsumi, H. Ishibashi, Synlett 2007, 658-660.
23. M. Benltifa, S. Vidal, D. Gueyrard, P. G. Goekjian, M. Msaddek, J.-P. Praly, Tetrahedron Lett. 2006, 47, 6143-6147
24. Altenbach, H.-J. ; Kottenhahn, M. ; Vogt, A. ; Matthäus, M. ; Grundler, A. ; Hahn, M. a) DE 19533617 A1, 1997 and b) USP6018050, 2000
25. Vogt, A. ; Altenbach, H.-J. ; Kirschbaum, M. ; Hahn, M. G. ; Matthäus, M. S. P. ; Hermann, A. R. a) EP 976721, 2000 ; b) Chem. Abstr. 2000, 132, 108296J.
26. Bert L, Robert F, Denoroy L, Stoppini L, Renaud B. J Chromatogr. A, 1996, 755, 99-111.
27. Bert L, Parrot S, Robert F, Desvignes C, Denoroy L, Suaud-Chagny MF, Renaud B. Neuropharmacology, 2002, 43, 825-835.
28. Parrot S, Bert L, Mouly-Badina L, Sauvinet V, Colussi-Mas J, Lambás-Señas L, Robert F, Bouilloux JP, Suaud-Chagny MF, Denoroy L, Renaud B. Cell Mol Neurobiol. 2003, 23, 793-804.
29. Sauvinet V, Parrot S, Benturquia N, Bravo-Moratón E, Renaud B, Denoroy L. Electrophoresis 2003, 24, 3187-3196.
30. Aharony, D. et al. (1993), Mol, Pharmacol, 44 : 356-363.
31. Ardati, A. et al. (1997), Mol. Pharmacol., 51 : 816-824.
32. Bloomquist, B.T. et al. (1998), Biochem. Biophys. Res. Commun., 243 : 474-479.
33. Bonhaus, D.W. et al. (1995), Brit. J. Pharmacol., 115 : 622-628.
34. Brown, G.B. (1986), J. Neurosci., 6 : 2064-2070.
35. Brown, P.J. et al. (1990), J. Biol. Chem., 265 : 17995-18004.
36. Buchan, K.W. et al. (1994), Brit. J. Pharmacol., 112 : 1251-1257.
37. Couvineau, A. et al. (1985), Biochem. J., 231 : 139-143.
38. Dorje, F. et al. (1991), J. Pharmacol. Exp. Ther., 256 : 727-733.
39. Fuhlendorff, J. et al. (1990), Proc. Natl. Acad. Sci. U.S.A., 87 : 182-186.
40. Grandy, D.K. et al. (1989), Proc. Natl. Acad. Sci. U.S.A., 86 :9762-9766.
41. Greengrass, P. and Bremner, R. (1979), Eur. J. Pharmacol., 55 :323-326.
42. Hope, A.G. et al. (1996), Brit. J. Pharmacol., 118 : 1237-1245.
43. Hoyer, D. et al. (1985), Eur. J. Pharmacol., 118 : 1-12.
44. Hugues, M. et al. (1982), J. Biol. Chem., 257 : 2762-2769.
45. Lewin, A.H. et al, (1989), Mol. Pharmacol., 35 : 189-194.
46. Luthin, D.R. et al. (1995), Mol. Pharmacol., 47 : 307-313.
47. Monaghan, D.T. and Cotman, C.W. (1982), Brain Res., 252 :91-100.
48. Monsma, F.J. et al. (1993), Mol. Pharmacol., 43 : 320-327.
49. Mulheron, J.G. et al. (1994), J. Biol. Chem., 269 : 12954-12962.
50. Murphy, D.E. et al. (1987), Neurochem. Res., 12 : 775-781.
51. Neote, K. et al. (1993), Cell, 72 : 415-425.
52. Pachotczyk, T. et al. (1991), Nature, 350 : 350-354.
53. Pristupa, Z.B. et al. (1994), Mol. Pharmacol., 45 : 125-135.
54. Rees, S. et al. (1994), FEBS Lett., 355 : 242-246.
55. Reynolds, I.J. et al. (1986), J. Pharmacol. Exp. Ther., 237 :731-738.
56. Salvatore, C.A. et al. (1993), Proc. Natl. Acad. Sci. U.S.A., 90 :10365-10369.
57. Schioth, H.B. et al. (1997), Neuropeptides, 31 : 565-571.
58. Shen, Y. et al. (1993), J. Biol. Chem., 268 : 18200-18204.
59. Baron, B.M. et al. (1996), J. Pharmacol. Exp. Ther., 279 : 62-68.
60. Sills, M.A. et al. (1991), Eur. J. Pharmacol., 192 : 19-24.
61. Sorensen, R.G. and Blaustein, M.P. (1989), Mol. Pharmacol., 36 :689-698.
62. Speth, R.C. et al. (1979), Life Sci., 24 : 351-358.
63. Townsend-Nicholson, A. and Schofield, P.R. (1994), J. Biol.Chem., 269 : 2373-2376.
64. Tsuji, A. et al. (1988), Antimicrob. Agents Chemother., 32 :190-194.
65. Uhlen, S. and Wikberg, J.E. (1991), Pharmacol. Toxicol., 69 :341-350 ; 257.
66. Vignon, J. et al. (1986), Brain Res., 378 :133-141.
67. Vita, N. et al. (1993), FEBS Lett., 317 : 139-142.
68. Wang, J.B. et al. (1994), FEBS Lett., 338 : 217-222.
69. White, J.R. et al. (1998), J. Biol. Chem., 273 : 10095-10098.
70. Zhou, Q.Y. et al. (1990), Nature, 347 : 76-80.
71. Tahara, A. et al. (1998), Brit. J. Pharmacol., 125 : 1463-1470.
72. Pruneau, D. et al. (1998), Brit. J. Pharmacol., 125 : 365-372.
73. Wieland, H. A. et al. (1995), J. Pharmacol. Exp. Ther., 275 :143-149.
74. Smit, M.J. et al. (1996), Brit. J. Pharmacol., 117 : 1071-1080.
75. Simonin, F. et al. (1994), Mol. Pharmacol., 46 :1015-1021.
76. Leurs, R. et al. (1994), Brit. J. Pharmacol., 112 : 847-854.
77. Peralta, E. G. et al. (1987), Embo. J., 6 : 3923-3929.
78. Levin, M.C. et al. (2002), J. Biol.Chem., 277 : 30429-30435.
79. Bignon, E. et al. (1999), J. Pharmacol. Exp. Ther. 289 : 742-751.
80. Tatsumi, M. et al. (1999), Eur. J. Pharmacol., 368 : 277-283.
81. Choi, D.S. et al. (1994), FEBS Lett., 352 : 393-399.
82. Witt-Enderby, P.A. and Dubocovich, M.L. (1996), Mol. Pharmacol.,50 : :166-174.
83. Rinaldi-Carmona, M. et al. (1996), J. PharmacoL Exp. Ther., 278 :871-878.
84. Sarau, H.M. et al. (1997), J. Pharmacol. Exp. Ther., 281 :1303-1311.
85. Meng, F. et al. (1993), Proc. Natl. Acad. Sci. U.S.A.., 90 :9954-9958.
86. Le, M.T. et al. (2005), Eur. J. Pharmacol., 513 : 35-45.
87. Abramovitz, M. et al. (2000), Biochem, Biophys. Acta., 1483 :285-293.
88. Joseph, S.S. et al. (2004), Naun.-Sch. Arch. Pharm., 369 :525-532.
89. Morgese MG et al. (2009) Neurochemical changes in the striatum of dyskinetic rats after administration of the cannabinoid agonist WIN55,212-2. Neurochem Int., 54(1):56-64.
90. Polissidis A. et al. (2013) Int J Neuropsychopharmacol., 16(2):393-403.
91. García-Arencibia M et al., (2008) Neurosci Lett. 438(1):10-3.
92. Berger C et al., (2004) J Neurochem. 88(5):1159-67.
93. Leweke et al (2012) Translational Psychiatry, 2, e94.
94. Lacrosse and Olive (2013) Neuropeptide Systems and Schizophrenia,CNS NeurolDisord Drug Targets, [Epub ahead of print]
95. Adam et al., (2012) Bioorganic & Medicinal Chemistry Letters.22:2932-2937.
96. Pintor A et al., (2006) Neuropharmacology, 51(5):1004-12.
97. Kirilly et al (2012) Acta Physiol., 1-20.
98. Thakur et al (2009) Expert Opin.Ther Patents, 19(12):1647-1673.
99. Strazielle N, et al. J Neurosci. 1999 Aug 1;19(15):6275-89.
100. Strazielle N, et al. AIDS. 2003 Jul 4;17(10):1473-85.
101. Strazielle N, et al. Methods Mol Med. 2003;89:291-304.
102. Teerlink T, et al. Anal Biochem. 2002 Apr 15;303(2):131-7.
103. Hermanson and Marnett (2011) Cancer Metastasis Rev., 30:599-612.
104. Alen et al (2012) Vitam.Horm., 92:165-96.
105. Robert W. Cowan, et al. Frontiers in Endocrinology (2012), July, Vol. 3 Article 89, doi : 10.3389/fendo.2012.00089.

## Revendications

1. Dérivés de pyrrolidine de formule (I) : 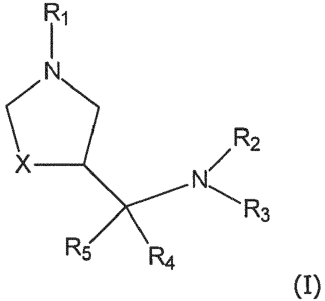 dans laquelle :
- R₁ représente un atome d'hydrogène, un groupe (C₁-C₆)alkyle ou C(O)(C₁-C₆)alkyle,
- R₂ et R₃ identiques ou différents, représentent, chacun indépendamment l'un de l'autre, un atome d'hydrogène ou un groupe (C₁-C₆)alkyle ; ou bien R₂=H et R₃= C(O)(C₁-C₆)alkyle,
- R₄ représente -COOH, -CN, -COORₐ, -C(=NOH)NH₂, -CH₂NH₂, -CH₂OH, -C(O)NH₂, -C(O)NHRₐ ou -COSRₐ, avec Rₐ qui représente un groupe (C₁-C₆)alkyle, ou bien R₄ représente un groupe tétrazole ou 1,2,4-oxadiazole,
- R₅ représente un atome d'hydrogène ou un groupe (C₁-C₆)alkyle,
- X représente -C(O)- ou -CHR_{b}-, avec R_{b} qui représente -OR_{c} ou -OC(O)R_{c}, R_{c} représentant un atome d'hydrogène ou un groupe (C₁-C₆)alkyle,
éventuellement sous une forme zwitterionique,
sous la forme d'un isomère optique pur, ou d'un mélange d'isomères optiques selon toutes proportions, ou sous une forme enrichie en un isomère optique, ainsi que leurs sels pharmaceutiquement acceptables, solvats ou hydrates.

2. Dérivés de pyrrolidine selon la revendication 1 **caractérisés en ce que** X représente -CHR_{b}, avec R_{b} tel que défini à la revendication 1.

3. Dérivés de pyrrolidine selon la revendication 1 ou 2 **caractérisés en ce qu'**il répond à la formule (Ia) : 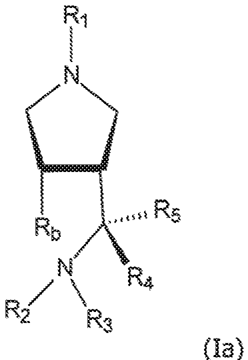 dans laquelle R₁, R₂, R₃, R₄, R₅ et R_{b} sont tels que définis à la revendication 1, ainsi que leurs sels pharmaceutiquement acceptables, solvats ou hydrates.

4. Dérivés de pyrrolidine selon la revendication 1 ou 2 **caractérisés en ce qu'**il répond à la formule (Ib) : 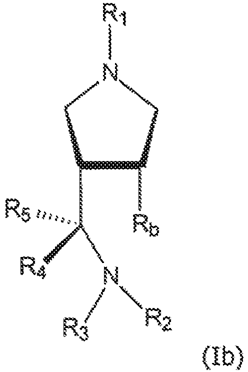 dans laquelle R₁, R₂, R₃, R₄, R₅ et R_{b} sont tels que définis à la revendication 1, ainsi que leurs sels pharmaceutiquement acceptables, solvats ou hydrates.

5. Dérivés de pyrrolidine selon l'une des revendications 2 à 4 **caractérisés en ce que** R_{b}=OH.

6. Dérivés de pyrrolidine selon l'une des revendications précédentes **caractérisés en ce que** R₁ est un atome d'hydrogène.

7. Dérivés de pyrrolidine selon l'une des revendications précédentes **caractérisés en ce que** R₂=H et R₃ représente un atome d'hydrogène ou un groupe méthyle ou -C(O)CH₃.

8. Dérivés de pyrrolidine selon l'une des revendications précédentes **caractérisés en ce que** R₄ représente -COOH, -C(O)NHCH₃ ou -CH₂NH₂.

9. Dérivés de pyrrolidine selon l'une des revendications précédentes **caractérisés en ce que** R₅ est un atome d'hydrogène.

10. Dérivés de pyrrolidine selon l'une des revendications précédentes **caractérisés en ce qu'**il se présente sous une forme salifiée ou zwitterionique comportant un ammonium quaternaire.

11. Dérivés de pyrrolidine selon la revendication 1 choisis parmi :
- l'acide α-amino-(4-hydroxy-pyrrolidin-3-yl)acétique (α*S*,3*R*,4*S*), composé **(I.1)** 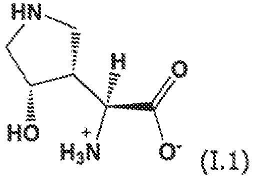
- l'acide α-amino-(4-hydroxy-pyrrolidin-3-yl)acétique (α*R*,3*S*,4*R*), composé (1.2) 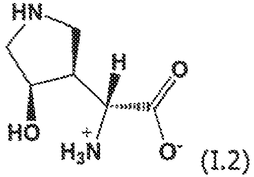
- l'α-acétamido-(4-hydroxy-pyrrolidin-3-yl)-*N*-méthylacétamide (α*R*,3*S*,4*R*), **composé (I.3)** 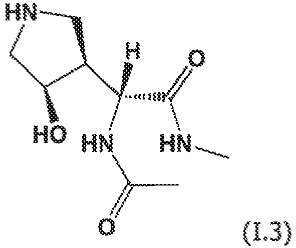
- l'α-amino-(4-hydroxy-pyrrolidin-3-yl)-*N*-méthylacétamide (α*S*,3*R*,4*S*), **composé (1.4)** 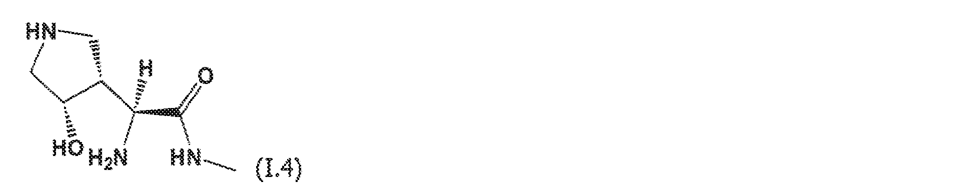
- l'acide α-*N*-méthylamino-(4-hydroxy-pyrrolidin-3-yl) acétique, 3,4-*cis*, **composé (1.5)** 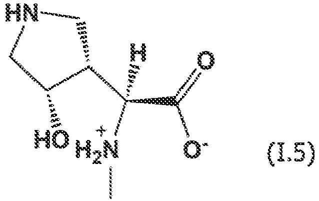
- la 3-(1,2-diaminoéthyl)-4-hydroxy-pyrrolidine 3,4-*cis*, composé (1.6) 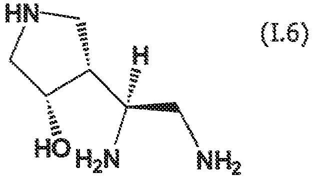 ainsi que leurs sels pharmaceutiquement acceptables, solvats ou hydrates.

12. Dérivés de pyrrolidine selon l'une des revendications précédentes pour leur utilisation en tant que médicament.

13. Dérivés de pyrrolidine selon l'une des revendications 1 à 11 pour leur utilisation dans le traitement de l'épilepsie, de l'ischémie, d'une maladie neurodégénérative telle que la maladie de Parkinson ou la chorée de Huntington, la sclérose en plaques, la maladie de Devic, la maladie d'Alzheimer, d'une maladie psychiatrique telle que la schizophrénie, la dépression, l'addiction, l'allodynie ou l'hyperalgie.

14. Dérivés de pyrrolidine selon l'une des revendications 1 à 11 pour leur utilisation dans le traitement de la maladie de Parkinson, de l'épilepsie, d'un trouble addictif ou de l'ischémie.

15. Dérivés de pyrrolidine selon l'une des revendications 1 à 11 pour leur utilisation dans le traitement de la douleur, d'un cancer d'un organe ou tissu périphérique, de l'obésité, d'un trouble squeletto-musculaire, d'une maladie cardio-vasculaire ou d'une maladie des voies digestives ou pour le contrôle de la prise alimentaire.

16. Compositions pharmaceutiques contenant un dérivé de pyrrolidine selon l'une des revendications 1 à 11, avec au moins un excipient pharmaceutiquement acceptable.

## Patentansprüche

1. Pyrrolidin-Derivate der Formel (I): 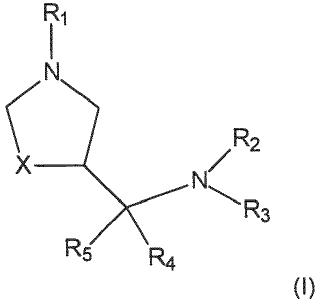 in der:
- R₁ ein Wasserstoffatom, eine (C₁-C₆)-Alkyl- oder C(0)(C₁-C₆)-Alkylgruppe darstellt,
- R₂ und R₃, die identisch oder unterschiedlich sind, jeweils unabhängig voneinander ein Wasserstoffatom oder eine (C₁-C₆)-Alkylgruppe darstellen; oder aber R₂=H und R₃=C(0)(C₁-C₆)-Alkyl,
- R₄ -COOH, -CN, -COORₐ, -C(=NOH)NH₂, -CH₂NH₂, -CH₂OH, -C(O)NH₂, -C(O)NHRₐ oder -COSRₐ darstellt, wobei Rₐ eine (C₁-C₆)-Alkylgruppe darstellt oder aber R₄ eine Tetrazol- oder 1,2,4-Oxadiazolgruppe darstellt,
- R₅ ein Wasserstoffatom oder eine (C₁-C₆)-Alkylgruppe darstellt,
- X -C(O)- oder -CHR_{b}- darstellt, wobei R_{b} -OR_{c} oder -OC(O)R_{c} darstellt, wobei R_{c} ein Wasserstoffatom oder eine (C₁-C₆)-Alkylgruppe darstellt, eventuell in einer zwitterionischen Form,
in Form eines reinen optischen Isomers oder einer Mischung aus optischen Isomeren in allen Verhältnissen, oder in einer mit einem optischen Isomer angereicherten Form, sowie deren pharmazeutisch akzeptable Salze, Solvate oder Hydrate.

2. Pyrrolidin-Derivate nach Anspruch 1, **dadurch gekennzeichnet, dass** X -CHR_{b}, darstellt, wobei R_{b} so ist wie in Anspruch 1 definiert.

3. Pyrrolidin-Derivate nach Anspruch 1 oder 2, **dadurch gekennzeichnet, dass** es der Formel (la) entspricht: 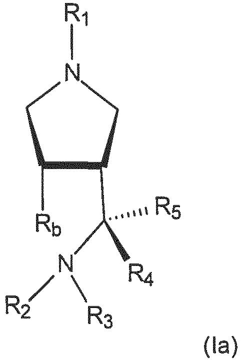 in der R₁, R₂, R₃, R₄, R₅ und R_{b} so sind wie in Anspruch 1 definiert, sowie deren pharmazeutisch akzeptable Salze, Solvate oder Hydrate.

4. Pyrrolidin-Derivate nach Anspruch 1 oder 2, **dadurch gekennzeichnet, dass** es der Formel (Ib) entspricht: 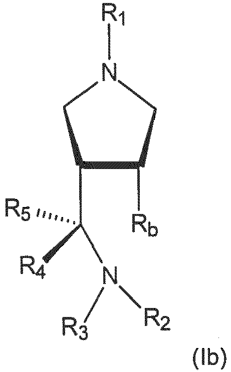 in der R₁, R₂, R₃, R₄, R₅ und R_{b} so sind wie in Anspruch 1 definiert, sowie deren pharmazeutisch akzeptable Salze, Solvate oder Hydrate.

5. Pyrrolidin-Derivate nach einem der Ansprüche 2 bis 4, **dadurch gekennzeichnet, dass** R_{b}=OH.

6. Pyrrolidin-Derivate nach einem der vorhergehenden Ansprüche, **dadurch gekennzeichnet, dass** R₁ ein Wasserstoffatom ist.

7. Pyrrolidin-Derivate nach einem der vorhergehenden Ansprüche, **dadurch gekennzeichnet, dass** R₂=H und R₃ ein Wasserstoffatom oder eine Methyl- oder - (C(O)CH₃-Gruppe darstellt.

8. Pyrrolidin-Derivate nach einem der vorhergehenden Ansprüche, **dadurch gekennzeichnet, dass** R₄ -COOH, -C(O)NHCH₃ oder -CH₂NH₂ darstellt.

9. Pyrrolidin-Derivate nach einem der vorhergehenden Ansprüche, **dadurch gekennzeichnet, dass** R₅ ein Wasserstoffatom ist.

10. Pyrrolidin-Derivate nach einem der vorhergehenden Ansprüche, **dadurch gekennzeichnet, dass** es in einer Salz- oder zwitterionischen Form mit einem quaternären Ammonium vorliegt.

11. Pyrrolidin-Derivate nach Anspruch 1, die ausgewählt sind aus:
- α-Amino-(4-hydroxy-pyrrolidin-3-yl) essigsäure (α*S*,3*R*,4*S*), Verbindung (I.1) 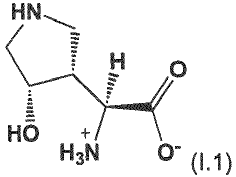
- α-Amino-(4-hydroxy-pyrrolidin-3-yl) essigsäure (α*R*,3*S*,4*R*), Verbindung (I.2) 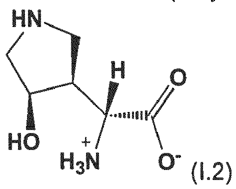
- α-Acetamid-(4-hydroxy-pyrrolidin-3-yl)-*N*-methylacetamid (α*R*,3*S*,4*R*), Verbindung (I.3) 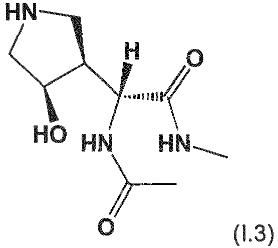
- α-Amino-(4-hydroxy-pyrrolidin-3-yl)-*N*-methylacetamid (α*S*,3*R*,4*S*), Verbindung (I.4) 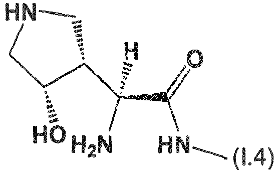
- α-*N*-methylamino-(4-hydroxy-pyrrolidin-3-yl) essigsäure, 3,4-*cis*, Verbindung (1.5) 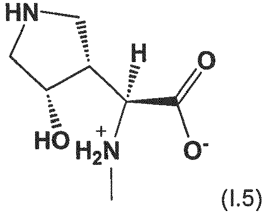
- 3-(1,2-diaminoethyl)-4-hydroxy-pyrrolidin 3,4-*cis*, Verbindung (I.6) 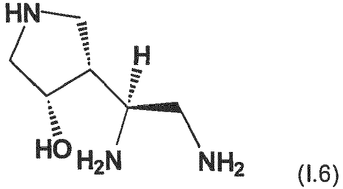 sowie deren pharmazeutisch akzeptable Salze, Solvate oder Hydrate.

12. Pyrrolidin-Derivate nach einem der vorhergehenden Ansprüche, für ihre Verwendung als Medikament.

13. Pyrrolidin-Derivate nach einem der Ansprüche 1 bis 11, für ihre Verwendung bei der Behandlung von Epilepsie, Ischämie, einer neurodegenerativen Erkrankung wie der Parkinson-Krankheit oder der Huntingtonschen Chorea, von Multiple Sklerose, der Devic-Erkrankung, der Alzheimer-Krankheit, einer psychiatrischen Erkrankung wie Schizophrenie, Depression, Sucht, Allodynie oder Hyperalgesie.

14. Pyrrolidin-Derivate nach einem der Ansprüche 1 bis 11, für ihre Verwendung bei der Behandlung der Parkinson-Krankheit, von Epilepsie, einer Suchtbeschwerde oder von Ischämie.

15. Pyrrolidin-Derivate nach einem der Ansprüche 1 bis 11, für ihre Verwendung bei der Behandlung von Schmerz, eines Krebses eines Organs oder peripheren Gewebes, von Fettleibigkeit, einer Skelett- und Muskelerkrankung, einer Herz-Kreislauf-Erkrankung oder einer Erkrankung des Verdauungstraktes oder zur Kontrolle der Nahrungszufuhr.

16. Pharmazeutische Zusammensetzungen, die ein Pyrrolidin-Derivat nach einem der Ansprüche 1 bis 11 enthalten, mit wenigstens einem pharmazeutisch akzeptablen Hilfsstoff.

## Claims

1. Pyrrolidine derivatives of formula (I): 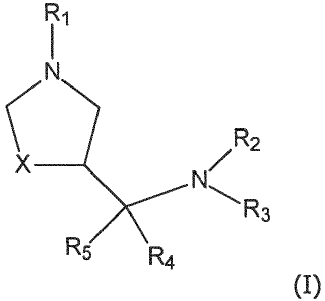 wherein:
- R₁ represents a hydrogen atom, a (C₁-C₆)alkyl or C(O)(C₁-C₆)alkyl group,
- R₂ and R₃, either identical or different, each represent independently of each other, a hydrogen atom or a (C₁-C₆)alkyl group; or else R₂=H and R₃= C(O)(C₁-C₆)alkyl,
- R₄ represents -COOH, -CN, -COORₐ, -C(=NOH)NH₂, -CH₂NH₂,-CH₂OH -C(O)NH₂, -C(O)NHRₐ or -COSRₐ, with Rₐ which represents a (C₁-C₆)alkyl group, or else R₄ represents a tetrazole or 1,2,4-oxadiazole group,
- R₅ represents a hydrogen atom or a (C₁-C₆)alkyl group,
- X represents -C(O)- or -CHR_{b}-, with R_{b} which represents -OR_{c} or -OC(O)R_{c}, R_{c} representing a hydrogen atom or a (C₁-C₆)alkyl group,
optionally in a zwitterionic form,
as a pure optical isomer, or a mixture of optical isomers in any proportions, or in a form enriched with an optical isomer, as well as their pharmaceutically acceptable salts, solvates or hydrates.

2. The pyrrolidine derivatives according to claim 1 **characterized in that** X represents -CHR_{b}, with R_{b} as defined in claim 1.

3. The pyrrolidine derivatives according to claim 1 or 2 **characterized in that** it fits the formula (Ia): 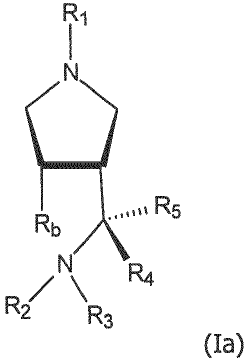 wherein R₁, R₂, R₃, R₄, R₅ and R_{b} are such as defined in claim 1, as well as their pharmaceutically acceptable salts, solvates or hydrates.

4. The pyrrolidine derivatives according to claim 1 or 2 **characterized in that** it fits the formula (Ib): 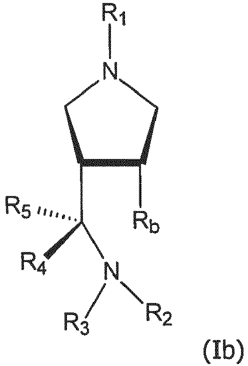 wherein R₁, R₂, R₃, R₄, R₅ and R_{b} are as defined in claim 1, as well as their pharmaceutically acceptable salts, solvates or hydrates.

5. The pyrrolidine derivatives according to one of claims 2 to 4, **characterized in that** R_{b}=OH.

6. The pyrrolidine derivatives according to one of the preceding claims, **characterized in that** R₁ is a hydrogen atom.

7. The pyrrolidine derivatives according to one of the preceding claims, **characterized in that** R₂=H and R₃ represents a hydrogen atom or a methyl or -C(O)CH₃ group.

8. The pyrrolidine derivatives according to one of the preceding claims, **characterized in that** R₄ represents -COOH, -C(O)NHCH₃ or -CH₂NH₂.

9. The pyrrolidine derivatives according to one of the preceding claims, **characterized in that** R₅ is a hydrogen atom.

10. The pyrrolidine derivatives according to one of the preceding claims, **characterized in that** it appears in a salified or zwitterionic form including a quaternary ammonium.

11. The pyrrolidine derivatives according to claim 1 selected from:
- (α*S*,3*R*,4*S*) α-amino-(4-hydroxy-pyrrolidin-3-yl)acetic acid, **compound (I.1)** 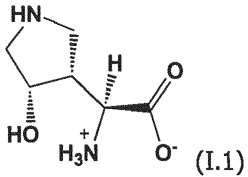
- (α*R*,3*S*,4*R*) α-amino-(4-hydroxy-pyrrolidin-3-yl)acetic acid, **compound (I.2)** 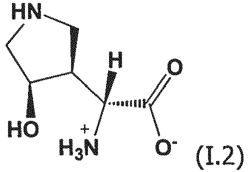
- (α*R*,3*S*,4*R*) α-acetamido-(4-hydroxy-pyrrolidin-3-yl)-*N*-methylacetamide, **compound (I.3)** 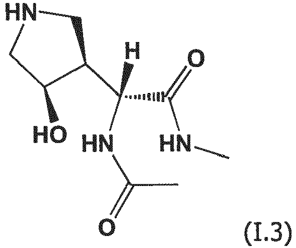
- (α*S*,3*R*,4*S*) α-amino-(4-hydroxy-pyrrolidin-3-yl)-*N*-methylacetamide, **compound** (I.**4**) 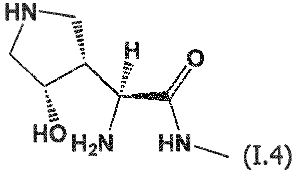
- 3,4-*cis* α-*N*-methylamino-(4-hydroxy-pyrrolidin-3-yl) acetic acid, **compound (1.5)** 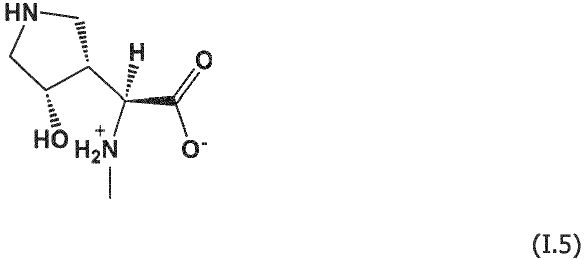
- 3,4-*cis* 3-(1,2-diaminoethyl)-4-hydroxy-pyrrolidine, compound (1.6) 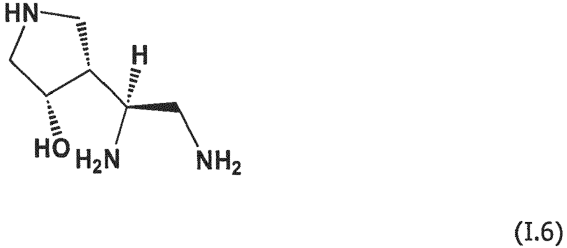 as well as their pharmaceutically acceptable salts, solvates or hydrates.

12. The pyrrolidine derivatives according to one of the preceding claims for their use as a drug.

13. The pyrrolidine derivatives according to one of the claims 1 to 11 for their use in the treatment of epilepsy, ischemia, a neurodegenerative disease such as Parkinson's disease or Huntington's chorea, multiple sclerosis, Devic's disease, Alzheimer's disease, a psychiatric disease such as schizophrenia, depression, addiction, allodynia or hyperalgia.

14. The pyrrolidine derivatives according to one of the claims 1 to 11 for their use in the treatment of Parkinson's disease, epilepsy, addictive disorder or ischemia.

15. The pyrrolidine derivatives according to one of the claims 1 to 11 for their use in the treatment of pain, cancer of an organ or peripheral tissue, obesity, musculoskeletal disorder, cardiovascular disease or a disease of the digestive tracts or for controlling food intake.

16. Pharmaceutical compositions containing a derivative of pyrrolidine according to one of the claims 1 to 11, with at least one pharmaceutically acceptable excipient.
